(19)

**Europäisches Patentamt**

**European Patent Office**

**Office européen des brevets**

(11) **EP 4 190 227 B1**

(12) **EUROPEAN PATENT SPECIFICATION**

(45) Date of publication and mention
of the grant of the patent:
**03.09.2025 Bulletin 2025/36**

(21) Application number: **21212048.9**

(22) Date of filing: **02.12.2021**

(51) International Patent Classification (IPC):
*A61B 5/00* (2006.01)    *A61B 5/053* (2021.01)
*A61B 5/02* (2006.01)

(52) Cooperative Patent Classification (CPC):
**A61B 5/7264; A61B 5/02014; A61B 5/02028;**
**A61B 5/053; A61B 5/7242; A61B 5/7246**

(54) **METHOD AND DEVICE FOR DETERMINING AN AORTIC STATE**

VERFAHREN UND VORRICHTUNG ZUR BESTIMMUNG EINES AORTENZUSTANDS

PROCÉDÉ ET DISPOSITIF PERMETTANT DE DÉTERMINER UN ÉTAT AORTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**07.06.2023 Bulletin 2023/23**

(73) Proprietor: **arterioscope FlexCo
8010 Graz (AT)**

(72) Inventors:
• **RANFTL, Sascha
8010 Graz (AT)**
• **BADELI, Vahid
8042 Graz (AT)**

(74) Representative: **Weiser Voith Gugler
Patentanwälte Partnerschaft
Kopfgasse 7
1130 Wien (AT)**

(56) References cited:
• **BADELI V. ET AL: "Electrode positioning to
investigate the changes of the thoracic
bioimpedance caused by aortic dissection - a
simulation study", JOURNAL OF ELECTRICAL
BIOIMPEDANCE, vol. 11, no. 1, 15 June 2020
(2020-06-15), pages 38 - 48, XP055921631, ISSN:
1891-5469, DOI: 10.2478/joeb-2020-0007**
• **PICHLER CHRISTOF ET AL: "Bayesian source
separation of electrical bioimpedance signals",
BIOMEDICAL SIGNAL PROCESSING AND
CONTROL, ELSEVIER, AMSTERDAM, NL, vol. 67,
16 March 2021 (2021-03-16), XP086536701, ISSN:
1746-8094, [retrieved on 20210316], DOI: 10.1016/
J.BSPC.2021.102541**
• **RANFTL SASCHA ET AL: "Bayesian Uncertainty
Quantification with Multi-Fidelity Data and
Gaussian Processes for Impedance
Cardiography of Aortic Dissection", ENTROPY,
vol. 22, no. 1, 31 December 2019 (2019-12-31),
pages 58, XP055921635, DOI: 10.3390/e22010058**

**Description**

The present invention relates to a method and a processing device for determining an aortic state of a patient.

Aortic pathologies, such as an aortic dissection, an aneurysm, an intramural hematoma, etc. are very serious and often even fatal when not treated properly and promptly. For proper treatment, the (pathological) aortic state has to be determined accurately. State of the art determination of whether a patient's aorta is in a healthy or in a (specific) pathologic state is performed on the basis of chest X-rays, computed tomography (CT), magnetic resonance imaging (MRI), which require expensive, large and bulky equipment, or on the basis of ultrasound imaging, the handling of which requires particular professional skills.

For more elementary measurements, e.g., of the heart rate or stroke volume, etc., impedance cardiography (ICG) has proven to be easy and reliable. In ICG, a pair of sensor electrodes is placed at specific spots on the patient's thorax defining a measuring segment therebetween; moreover, a pair of injector electrodes is placed at specific spots on the patient's thorax, each behind a different one of the sensor electrodes, outside the measuring segment. This configuration of a set of four electrodes is called a "tetra-polar" electrode configuration. Subsequently, an alternating current is injected along the thorax by means of the outer injector electrodes. A significant portion of the current passes through the aorta, and a respective voltage is captured by means of the inner sensor electrodes. The impedance is sensed over time by employing the known relation $Z = U/I$, wherein $Z$, $U$, and $I$ denote the sensed impedance, the captured voltage, and the injected current, respectively. The sensed impedance, which can, e.g., be the absolute value of the impedance over one or more cardiac cycles, is subsequently analysed by a physician.

ICG electrodes can be easily placed on the patient's thorax such that the results of ICG measurements are quickly available. However, state of the art ICG measurements do not allow to accurately, reliably and easily determine the aortic state therefrom for the following reasons.

Firstly, it is particularly difficult per se to distinguish the sensed impedances of healthy aortas from pathologic aortas, not to mention a distinction between different pathologic states, e.g., between aortas showing an ascending aortic dissection, a descending aortic dissection, an aneurysm, an intramural haematoma, a penetrating atherosclerotic ulcer, or the like.

Secondly, in case of a pathologic aortic state, the sensitivity of the ICG electrodes strongly depends on the (unknown) position of a pathological change in the patient's aorta relative to the placement spots of the ICG electrodes. A low sensitivity to a pathological aortic change impedes a distinction between different aortic states. In the worst case, a pathological aortic change residing at an inauspicious position relative to the chosen spots of the ICG electrodes may not even significantly influence the impedance sensed over time and may, thus, result in a faulty assessment of the patient's aortic state and, consequently, even in a medical malpractice.

In V. Badeli et. al "Electrode positioning to investigate the changes of the thoracic bioimpedance caused by aortic dissection - a simulation study", Journal of electical bioimpedance, vol 11, pp. 38 - 48, 2020, positioning of the set ICG electrodes is investigated from polynomial chaos expansion (PCE) metamodels of impedances for different aortic states and different electrode positions. The metamodels are parameterised by quantified impedances of simulated thorax models.

A decomposition of the sensed impedance into different source terms, e.g. pulsatory and respiratory sources, by means of Bayesian probability theory is described in C. Pichler et. al "Bayesian source separation of electrical bioimpedance signals", Biomedical Signal Processing and Control, vol 67, article number 102541, 2021.

It is an object of the present invention to provide a method and a processing device which can determine an aortic state of a patient, and a measuring system and method which can measure the aortic state of the patient, in each case reliably, accurately and automatically.

In a first aspect, this object is achieved with a method for determining an aortic state of a patient based on sensed impedances by means of a processing device which has a processor and a memory connected thereto, wherein each of said impedances has been sensed over time by one of at least two different sets of impedance cardiography, ICG, electrodes connectable to the processing device and configured to be placed in a tetra-polar electrode configuration at respective spots on the patient's thorax, the method comprising:

receiving in said memory, from said sets of ICG electrodes said impedances;
prior to, during, or after said receiving, storing in said memory, for each of a predetermined set of aortic states and each set of ICG electrodes, a surrogate model generated for a plurality of reference objects having that aortic state, which surrogate model describes a reference impedance over time;
computing, by means of the processor, for each aortic state of the set, a similarity value from a product of probability functions each of which depends on a difference between the sensed impedance for a respective set of ICG electrodes and the reference impedance for that aortic state and the same set of ICG electrodes; and
determining, by means of the processor, the aortic state with the highest computed similarity value as the patient's aortic state.

The invention automatically combines impedances sensed over time by the different sets of ICG electrodes into one aortic state determination. By using at least two different sets of ICG electrodes in tetra-polar electrode configuration placed at different spots on the patient's thorax, inauspicious positions of potential pathological aortic changes of the patient are reliably precluded. Inaccuracies and incorrect analyses are greatly reduced or even eliminated. As applicant's research has shown, employing probability functions is particularly suited for efficiently computing the similarity values. An undue increase of computational complexity with the number of sensed impedances can be avoided.

The similarity values accurately indicate a similarity between the sensed and reference impedances and, thus, the respective aortic state. Moreover, the similarity values are not only suitable to determine the aortic state but also - when comparing their magnitudes - to estimate the certainty of the determined aortic state. For example, when the difference of similarity values of a healthy state, which has been determined as the patient's aortic state, and a pathologic state is rather small, precautionary measures such as a further measurement may be taken.

The probability function may be a distribution function such as a log-normal distribution, a Pareto distribution, a gamma distribution, a Student's t-distribution, etc., each with its maximum corresponding to said difference between the sensed and reference impedances being zero.

In a favourable embodiment of the invention, each probability function is a Gaussian function according to

$$\frac{1}{\sqrt{2\pi\Delta_{i,j,k}^2}} \exp\left(-\frac{\left(Z_j(t_k)-f_{i,j}(t_k)\right)^2}{2\Delta_{i,j,k}^2}\right) \tag{1}$$

with

exp the exponential function,

$Z_j(t_k)$ the sensed impedance for the $j^{th}$ set of ICG electrodes at the $k^{th}$ sensing time,

$f_{i,j}(t_k)$ the reference impedance for the $i^{th}$ aortic state and the $j^{th}$ set of ICG electrodes at the $k^{th}$ sensing time, and

$\Delta_{i,j,k}$ an estimated uncertainty as to the sensing by the $j^{th}$ set of ICG electrodes and/or as to the reference impedance for the $i^{th}$ aortic state and the $j^{th}$ set of ICG electrodes at the $k^{th}$ sensing time.

The Gaussian function accounts for the estimated uncertainty with regard to the sensing and/or with regard to the reference impedance in a simple manner, which further increases the accuracy of the determination of the aortic state of the patient. Moreover, the product of exponential functions is particularly efficiently computed by the processor, e.g., by summing the exponents and evaluating a resulting single exponential function.

In a preferred embodiment of the invention, each reference impedance is an expansion of basis functions which depend on predefined aortic parameters of the aortic state, and said step of computing is performed by marginalising the product of probability functions with respect to the aortic parameters. Such an expansion takes aortic parameters into account, e.g., an outward bulging of parts of the aorta, mechanical parameters, e.g., a stiffness, of the aorta, a blood composition, or a position of a pathologic change, and, thus, renders the similarity value computation particularly accurate. While the values of these aortic parameters can be determined, e.g., measured, for the reference objects when generating the surrogate models, the aortic parameters of the patient need not be determined in separate time-consuming measurements. This is due to said marginalising, wherein each similarity value is computed as a marginal distribution by integrating, over the aortic parameters, the product of probability functions multiplied by a predetermined prior probability for the aortic parameters and for the aortic state. The prior probability, which indicates the probability for the values of the aortic parameters being present given the aortic state, may be accurately measured or efficiently estimated.

Advantageously, the aortic parameters of a healthy aortic state are a haematocrit level and a maximum radius of the true lumen. Favourably, the aortic parameters of a dissected aortic state are a maximum radius of the true lumen, a maximum radius of the false lumen, a haematocrit level, and a position of the false lumen relative to the true lumen. As applicant's research has shown, these aortic parameters can be significant for accurately describing the reference impedances of reference objects with a healthy and a pathologic aorta, respectively.

It is beneficial when, in said step of computing, each similarity value is computed according to

$$p(AS_i|\mathbf{Z}) = \frac{p(AS_i)}{Vol(\mathbf{R}_i)} \int_{\mathbf{R}_i} p(\mathbf{Z}|\mathbf{x}_i, AS_i) \, dV_{\mathbf{x}_i} \tag{2}$$

preferably according to

$$p(AS_i \mid \mathbf{Z}) = \frac{p(AS_i)}{Vol(\mathbf{R}_i)} \int_{\mathbf{R}_i} \prod_{\substack{j=1,\ldots,J \\ k=1,\ldots,K}} \frac{1}{\sqrt{2\pi\Delta_{i,j,k}^2}} \exp\left(-\frac{\left(Z_j(t_k) - f_{i,j}(\mathbf{x}_i, t_k)\right)^2}{2\Delta_{i,j,k}^2}\right) dV_{\mathbf{x}_i} \qquad (3)$$

with $p(AS_i \mid \mathbf{Z})$     the computed similarity value of the $i^{th}$ aortic state given the sensed impedances $\mathbf{Z}$,

$p(AS_i)$     a predetermined prior probability for the $i^{th}$ aortic state,

$\mathbf{R}_i$     a predetermined set of values of the aortic parameters $\mathbf{x}_i$ of the $i^{th}$ aortic state,

$Vol(\mathbf{R}_i)$     a hypervolume spanned by said predetermined set of values,

$p(\mathbf{Z} \mid \mathbf{x}_i, AS_i)$.     the product of probability functions,

$dV_{x_i}$     a hypervolume element,

$\int_{\mathbf{R}_i}$     an integration over the predetermined set of values,

$\prod_{\substack{j=1,\ldots,J \\ k=1,\ldots,K}}$     a product over all J sets of ICG electrodes and all K sensing times,

$Z_j(t_k)$     the sensed impedance for the $j^{th}$ set of ICG electrodes at the $k^{th}$ sensing time,

$f_{i,j}(\mathbf{x}_i, t_k)$     the reference impedance for the $i^{th}$ aortic state and the $j^{th}$ set of ICG electrodes at the $k^{th}$ sensing time in dependence on the aortic parameters $\mathbf{x}_i$ of the $i^{th}$ aortic state, and

$\Delta_{i,j,k}$     an estimated uncertainty as to the sensing by the $j^{th}$ set of ICG electrodes and/or as to the reference impedance for the $i^{th}$ aortic state and the $j^{th}$ set of ICG electrodes at the $k^{th}$ sensing time.

In this case, the marginalising is a particularly simple and efficient computation, which reduces the required computing power and/or time. In addition, this marginalising does not require a complex predetermination of a prior probability of the aortic parameters for the respective aortic state.

Advantageously, in said step of computing, each reference impedance is calculated by means of the processor from the surrogate model which describes the reference impedance by

$$f_{i,j}(\mathbf{x}_i, t_k) = f_{i,j}(\mathbf{x}_i; \mathbf{c}_i(t_k)) = \sum_{q=1,\ldots,Q} c_{i,j,q}(t_k) \cdot \phi_q(\mathbf{x}_i) \qquad (4)$$

with

$f_{i,j}(\mathbf{x}_i, t_k)$     the calculated reference impedance for the $i^{th}$ aortic state and the $j^{th}$ set of ICG electrodes at the $k^{th}$ sensing time in dependence on the aortic parameters $\mathbf{x}_i$ of the $i^{th}$ aortic state,

$\phi_q(\mathbf{x}_i)$     a multi-variate polynomial in the aortic parameters $\mathbf{x}_i$ of the $i^{th}$ aortic state, wherein the polynomials are indexed by q,

$c_{i,j,q}(t_k)$     an expansion coefficient for the $i^{th}$ aortic state, the $j^{th}$ set of ICG electrodes and the $q^{th}$ polynomial at the $k^{th}$ sensing time,

$\mathbf{c}_i$     the set of all expansion coefficients for the $i^{th}$ aortic state, and

$\sum_{q=1,\ldots,Q}$     a sum over all Q multi-variate polynomials.

With this expansion, a particularly accurate and fast measurement of the aortic state is achieved. Moreover, such an expansion in multi-variate polynomial basis functions is particularly simple and allows to calculate the reference impedances quickly and efficiently and, thus, requires only little processor resources.

In some embodiments, the surrogate models are generated in a step preceding the inventive method, e.g. from real world reference objects such as humans or animals. In a favourable variant, however, the reference objects are finite element method, FEM, models of thoraces and the method further comprises, prior to said step of storing, generating, by means of the processor, each surrogate model by:

determining, for each of the reference objects, the values of the aortic parameters of the aortic state of the reference object;

applying, for each set of ICG electrodes and each of the reference objects, the ICG electrodes on that reference object's thorax at the respective spots and quantifying, by means of the applied ICG electrodes, an impedance over time; and

calculating, by means of the processor from the determined values of the aortic parameters and the quantified impedances, the expansion coefficients.

In this variant, the steps of determining, applying and quantifying can be carried out by creating the FEM model thorax with desired values of the aortic parameters and simulating the resulting impedance over time for the respective spots of the ICG electrodes of each set. This can be done in a fast and accurate manner, which allows to quickly create a large set of reference objects for a desired range of aortic parameters and the respective quantified impedances.

The expansion coefficients may be calculated by the processor by regression or by fitting the surrogate models to the respective quantified impedances of the same set of ICG electrodes and the same aortic state. However, it is particularly beneficial when the expansion coefficients are calculated according to

$$c_{i,j,q}(t_k) = \sum_l \left( \left( \mathbf{U}_i^T \cdot \mathbf{U}_i \right)^{-1} \cdot \mathbf{U}_i^T \right)_{ql} Z_j^l(t_k) \qquad (5)$$

with

$c_{i,j,q}(t_k)$ the expansion coefficient for the $i^{th}$ aortic state, the $j^{th}$ set of ICG electrodes and the $q^{th}$ polynomial at the $k^{th}$ sensing time,

$\mathbf{U}_i = \phi_q(\mathbf{v}_i^l)$ a matrix for the $i^{th}$ aortic state formed by evaluating the $q^{th}$ multi-variate polynomial at the determined values $\mathbf{v}_i^l$ of the aortic parameters $\mathbf{x}_i$ of the $l^{th}$ reference object having the $i^{th}$ aortic state, and

$Z_j^l(t_k)$ the quantified impedance of the $l^{th}$ reference object in the $j^{th}$ set of ICG electrodes at the $k^{th}$ sensing time.

Surrogate models generated with these calculated expansion coefficients describe reference impedances which, on average, have the smallest error with respect to the underlying quantified impedances of the reference objects and, consequently, facilitate a particularly accurate determination of the aortic state.

The impedances are sensed in preliminary steps before the inventive method is carried out and provided to the processing device for determination of the aortic state.

In a second aspect, the invention provides for a processing device for determining an aortic state of a patient, the processing device having

a memory which is configured to receive, from at least two different sets of impedance cardiography, ICG, electrodes connectable to said processing device and configured to be placed in a tetra-polar electrode configuration at respective spots on the patient's thorax, an impedance sensed over time by that set of ICG electrodes, and to store, for each of a predetermined set of aortic states and each set of ICG electrodes, a surrogate model generated for a plurality of reference objects having that aortic state, which surrogate model describes a reference impedance over time; and

a processor connected to said memory and configured to compute, for each aortic state of the set, a similarity value from a product of probability functions each of which depends on a difference between the sensed impedance for a respective set of ICG electrodes and the reference impedance for that aortic state and the same set of ICG electrodes, and to determine the aortic state with the highest computed similarity value as the patient's aortic state.

With respect to the advantages and variants of the processing device reference is made to the abovementioned embodiments and variants of the method.

In a third aspect, the invention provides for a measuring system comprising the above-mentioned processing device and said at least two different sets of ICG electrodes, wherein each set of ICG electrodes is configured to be placed in the tetra-polar electrode configuration at the respective spots on the patient's thorax, to sense said impedance over time, and to transmit the sensed impedance to the memory.

Relating to further embodiments and variants of the measuring system and the advantages thereof, reference is made to the above statements on the method.

The invention shall now be described in further detail by means of exemplary embodiments thereof under reference to the enclosed drawings, in which:

Fig. 1 shows a measuring system according to the invention for measuring an aortic state of a patient in a schematic view;

Fig. 2 shows a method according to the invention carried out by the measuring system of Fig. 1 in a flow diagram;

Fig. 3 shows an impedance sensed over time by a set of impedance cardiography electrodes of the measuring system of Fig. 1 in a diagram over time;

Fig. 4 shows optional steps of a variant of the method of Fig. 2 in a flow diagram; and

Fig. 5 shows a finite element method (FEM) model of a thorax employed in the variant of Fig. 4 in an oblique perspective view from behind.

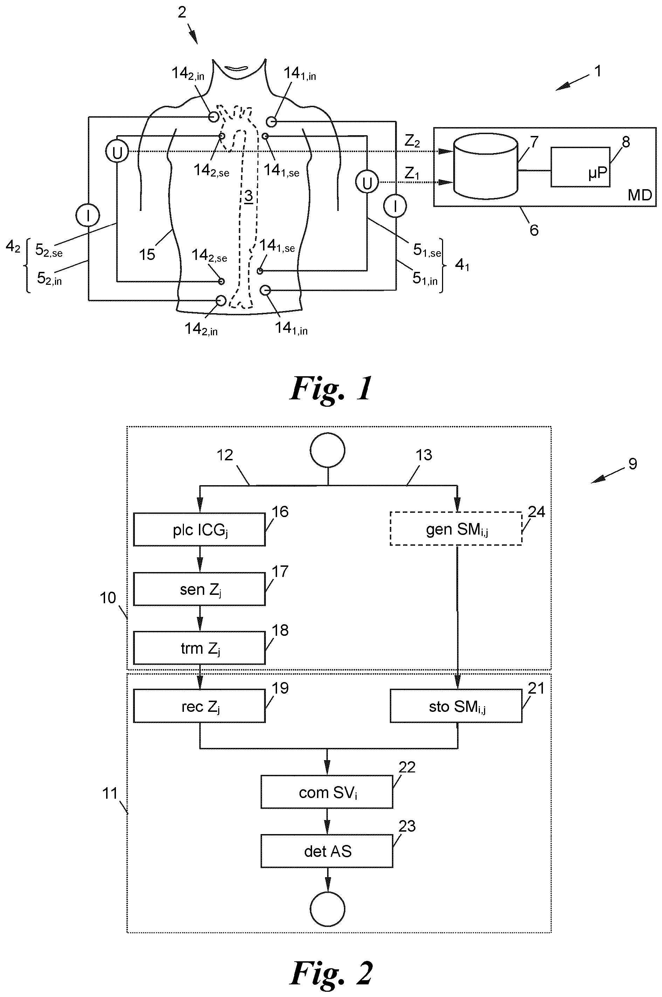

Fig. 1 shows a measuring system 1 which measures an aortic state of a patient 2. The patient 2 may be human or animal and the state of the patient's aorta 3 is one of a predetermined

set of two or more aortic states. The set contains a healthy state and one or more pathologic states, e.g., a state of an ascending aortic dissection, a state of a descending aortic dissection, an aneurysmal state, etc., as required.

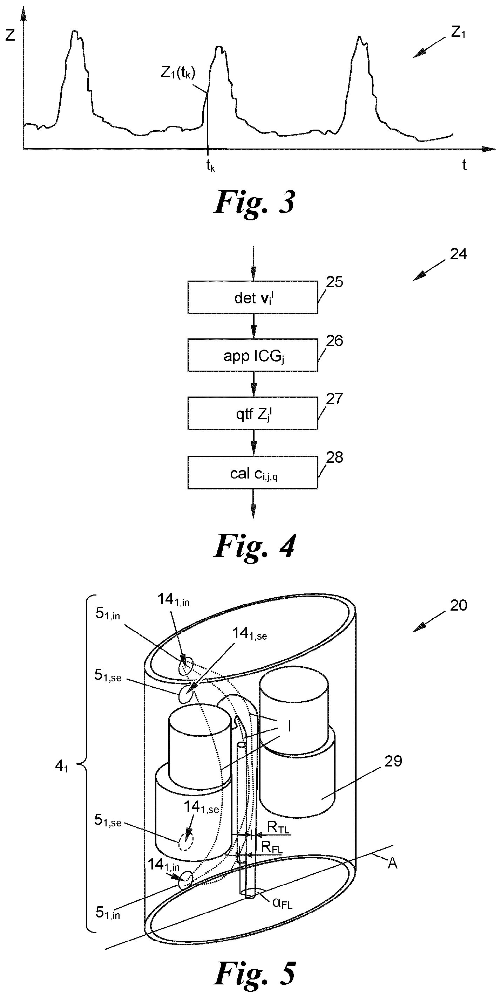

To measure the aortic state of the patient 2, the measuring system 1 comprises at least two different sets $4_1$, $4_2$, ... $4_J$, generally $4_j$, of impedance cardiography (ICG) electrodes $5_{1,in}$, $5_{1,se}$, $5_{2,in}$, $5_{2,se}$, ..., $5_{J,in}$, $5_{J,se}$, generally $5_{j,in}$, $5_{j,se}$, for sensing a respective impedance $Z_1$, $Z_2$, ..., $Z_J$, generally $Z_j$, over time (Fig. 3). The measuring system 1 also comprises a processing device 6 which determines the aortic state of the patient 2 based on the impedances $Z_j$ sensed over time by the ICG electrodes $5_{j,in}$, $5_{j,se}$, respectively. To this end, the processing device 6 has a memory 7 and a processor 8 connected to the memory 7.

With reference to Figs. 1 to 3, a method 9 carried out by the measuring system 1 for measuring the aortic state shall now be described.

As illustrated in Fig. 2, the method 9 is divided into two phases 10, 11, wherein the first phase 10 substantially comprises the sensing of the impedances $Z_j$ (branch 12). Surrogate models are provided and optionally generated in a branch 13, either in the first or in the second phase 10, 11 (here: in the first phase 10). The second phase 11 is directed at determining the patient's aortic state based on the sensed impedances $Z_j$ and the surrogate models. Taken in isolation, this phase 11 of determining constitutes a method on its own which is carried out by the processing device 6.

During the first phase 10 in the first branch 12, the ICG electrodes $5_{j,in}$, $5_{j,se}$ of each set $4_j$ are placed in a tetra-polar electrode configuration at respective spots $14_{j,in}$, $14_{j,se}$ on the patient's thorax 15 in step 16. To this end, each set $4_j$ of ICG electrodes $5_{j,in}$, $5_{j,se}$ (herein also: "electrode set" $4_j$) comprises a pair of sensor electrodes $5_{j,se}$ which is placed at specific sensing spots $14_{j,se}$ on the patient's thorax 15, forming a measuring segment therebetween, and a pair of injector electrodes $5_{j,in}$ which is placed at specific injecting spots $14_{j,in}$ on the patient's thorax 15, each behind a different one of the sensor electrodes $5_{j,se}$, outside the measuring segment. The specific spots $14_{j,in}$, $14_{j,se}$ are predetermined for each set $4_j$ of ICG electrodes $5_{j,in}$, $5_{j,se}$ and may be at the front, the side and/or the back of the thorax 15. It shall be mentioned that, for the sake of easier understanding, two sets $4_1$, $4_2$ of a total of eight ICG electrodes $5_{1,in}$, $5_{1,se}$, $5_{2,in}$, $5_{2,se}$ are identified in Fig. 1; however, the shown ICG electrodes $5_{1,in}$, $5_{1,se}$, $5_{2,in}$, $5_{2,se}$ can be deployed to form further electrode sets, e.g., an additional electrode set formed by the right injector electrodes $5_{1,in}$ and the left sensor electrodes $5_{2,se}$, or an additional electrode set formed by the respective upper ones of the left injector electrodes $5_{2,in}$ and of the left sensor electrodes $5_{2,se}$ and by the respective lower ones of the right injector electrodes $5_{1,in}$ and of the right sensor electrodes $5_{1,se}$, etc. Moreover, further sensor and/or injector electrodes $5_{j,in}$, $5_{j,se}$ forming one or more additional electrode sets $4_j$ may be placed on the patient's thorax 15. Generally speaking, any tetra-polar configuration of sensor and injector electrodes $5_{j,in}$, $5_{j,se}$ may be employed to form the electrode sets $4_j$. Hence, an electrode set $4_j$ may optionally even comprise more than two injector electrodes.

In step 17, a respective impedance $Z_j$ is sensed over time for each electrode set $4_j$ by the ICG electrodes $5_{j,in}$, $5_{j,se}$ of that electrode set $4_j$. This is achieved by injecting an alternating current I into the thorax 15 by means of the injector electrodes $5_{j,in}$ of the electrode set $4_j$ and by sensing a resulting voltage U and, together therewith, the impedance $Z_j$ using the relation $Z_j = U/I$ by means of the sensor electrodes $5_{j,se}$ of the electrode set $4_j$.

In the present method 9, the impedance $Z_j$ is either a complex value taking into account the phase between the current I and the voltage U, the modulus thereof, and/or the derivative of one of the two.

Fig. 3 shows an example for an impedance $Z_1$ that has been sensed over time (here: its modulus) by means of the first set $4_1$ of ICG electrodes $5_{1,inj}$, $5_{1,sens}$ over three cardiac cycles; at an exemplary sensing time $t_k$, the sensed impedance $Z_1$ is $Z_1(t_k)$.

Steps 16 and 17 may be carried out one after the other, i.e., by placing the ICG electrodes $5_{j,in}$, $5_{j,se}$ of all electrode sets $4_j$ on the patient's thorax 15 and then sensing the respective impedances $Z_j$ over time one after the other or even simultaneously, e.g., at respective different frequencies; alternatively, steps 16 and 17 may be intermeshed in that the ICG electrodes $5_{1,in}$, $5_{1,se}$ of a single electrode set $4_1$ and $4_2$, respectively, are initially placed at the spots $14_{1,in}$, $14_{1,se}$ on the thorax 15 forming the first electrode set $4_1$, and the first impedance $Z_1$ is then sensed over time, thereafter the same ICG electrodes $5_{1,in}$, $5_{1,se}$ are placed at different spots $14_{2,in}$, $14_{2,se}$ on the thorax 15, forming the second electrode set $4_2$, and the second impedance $Z_2$ is sensed over time (not shown), etc.

Coming back to Fig. 2, in step 18, for each set $4_j$ of ICG electrodes, the impedance $Z_j$ sensed by the electrode set $4_j$ is transmitted by means of the electrode set $4_j$ to the memory 7, either one after the other or as a packet of two or more impedances $Z_1$, $Z_2$, ..., e.g., via a wired or a wireless connection as known in the art.

In final step 19 of the first branch 12 which constitutes the starting point of the second phase (or method) 11, the

respective transmitted impedance $Z_j$ is received in memory 7 from each of the at least two different electrode sets $4_j$.

In the second branch 13, the processing device 6 is provided with a respective surrogate model for each of the predetermined sets of aortic states and for each set $4_j$ of ICG electrodes $5_{j,in}$, $5_{j,se}$. Each surrogate model is generated for a plurality of reference objects 20 (Fig. 5) having that aortic state and describes a reference impedance over time. Each reference impedance over time, thus, corresponds to a set $4_j$ of ICG electrodes $5_{j,in}$, $5_{j,se}$ placed at the respective abovementioned spots $14_{j,in}$, $14_{j,se}$.

The generation of the surrogate models will be explained in detail with respect to Figs. 4 and 5 further down.

In step 21 which is part of the second phase 11, the generated surrogate models are stored in the memory 7 and, thus, are accessible to the processor 8. Each surrogate model is stored in such a format that the processor 8 can determine the described reference impedance over time therefrom, e.g., as a parameterised function, as the reference impedances for respective sensing times $t_k$ in an array, or the like.

When the first and second branches 12, 13 have been passed, the processor 8 determines the aortic state of the patient 2 from the sensed impedances $Z_j$ and the reference impedances. To this end, the processor 8 computes, for each aortic state, a similarity value from a product of probability functions in step 22. In one variant, the similarity value is computed by multiplying the product with a predetermined prior probability for the respective aortic state; in another variant, which is described below, a more elaborate method is performed. Each probability function depends on a difference between the impedance $Z_j$ sensed by one set $4_j$ of ICG electrodes $5_{j,in}$, $5_{j,se}$ and the reference impedance for that aortic state and the same set $4_j$ of ICG electrodes $5_{j,in}$, $5_{j,se}$, i.e., the ICG electrodes $5_{j,in}$, $5_{j,se}$ placed (or applied, as described further below) at equivalent spots $14_{j,in}$, $14_{j,se}$ on the thorax 15 of the patient 2 and on the thorax of the reference objects 20, respectively. Consequently, each of the similarity values computed in step 22 is indicative of a similarity between the sensed impedances $Z_j$ and the reference impedances described by the surrogate model for the respective aortic state.

In a first variant of step 22, each probability function is taken at the respective sensing time $t_1, t_2, ..., t_K$, generally $t_k$, and depends on said difference according to

$$FP_{i,j} = FP_{i,j}(t_k) = FP_{i,j}(Z_j(t_k) - f_{i,j}(t_k)) \qquad (6a)$$

and the product is calculated as

$$p(\mathbf{Z}|AS_i) = \prod_{\substack{j=1,...,J \\ k=1,...,K}} FP_{i,j}(Z_j(t_k) - f_{i,j}(t_k)) . \qquad (6b)$$

with

$p(\mathbf{Z}|AS_i)$ the product of probability functions calculated from all sensed impedances $\mathbf{Z} = \{Z_1(t), Z_2(t), ..., Z_J(t)\}$ for the $i^{th}$ aortic state $AS_i$ (bold printing indicating a set),

$\displaystyle\prod_{\substack{j=1,...,J \\ k=1,...,K}}$ a product over all J sets of ICG electrodes and all K sensing times,

$FP_{i,j}$ the probability function for the $i^{th}$ aortic state $AS_i$ and the $j^{th}$ set $4_j$ of ICG electrodes $5_{j,in}$, $5_{j,se}$,

$Z_j(t_k)$ the sensed impedance for the $j^{th}$ set $4_j$ of ICG electrodes $5_{j,in}$, $5_{j,se}$ at the $k^{th}$ sensing time,

$f_{i,j}(t_k)$ the reference impedance for the $i^{th}$ aortic state $AS_i$ and the $j^{th}$ set $4_j$ of ICG electrodes $5_{j,in}$, $5_{j,se}$ at the $k^{th}$ sensing time $t_k$.

In a second variant of step 21, each probability function is taken for a respective time interval $\Delta t_k = t_{k+1} - t_k$, and depends on said difference, e.g., according to

$$FP_{i,j} = FP_{i,j}(\Delta t_k) = FP_{i,j}\left(\int_{t_k}^{t_{k+1}} (Z_j(t) - f_{i,j}(t)) \, dt\right), \qquad (7a)$$

with

$\int dt$ an integration over time,

and the product is calculated as

$$p(\mathbf{Z}|AS_i) = \prod_{\substack{j=1,...,J \\ k=1,...,K-1}} FP_{i,j}(\int_{t_k}^{t_{k+1}} (Z_j(t) - f_{i,j}(t))dt) . \qquad (7b)$$

The sizes of the time intervals $\Delta t_k$ may, e.g., be small (in the range of several μs), medium (in the range of several ms), or even large (in the range of, e.g., a cardiac cycle or of the whole sensing time). In further variants of this embodiment, instead of the integral employed in equations (7a) and (7b), another mathematical functional known in the art can be employed to map the difference over time to a scalar.

In further variants, the product is calculated as a weighted product, e.g., as

$$p(\mathbf{Z}|AS_i) = \prod_{j,k} FP_{i,j}(Z_j(t_k) - f_{i,j}(t_k)) \cdot w_{j,k} \qquad (8)$$

wherein at least one weight $w_{j,k}$ for the $j^{th}$ set $4_j$ of ICG electrodes and the $k^{th}$ sensing time $t_k$ has a value not equal to one, e.g., to take correlations into account.

Subsequent to step 22, in final step 23 of the method 9, the processor 8 determines the aortic state with the highest computed similarity value as the patient's aortic state. Therefor, the processor 8 may simply compare the computed similarity values. The determined patient's aortic state may then be output, e.g., on a display (not shown). Optionally, all computed similarity values may be output, e.g., to yield an indication for the certainty of each determined aortic state for reason of comparison.

Below, embodiments and variants of the step 22 of computing the similarity values shall be described.

Each probability function employed in step 22 is, e.g., a function having larger values the smaller the difference between the sensed and the reference impedances of the respective aortic state, i.e., between $Z_j$ and $f_{i,j}$, is. In an optional embodiment, each probability function is a Gaussian function according to

$$FP_{i,j}(t_k) = \frac{1}{\sqrt{2\pi\Delta_{i,j,k}^2}} \exp\left( -\frac{(Z_j(t_k) - f_{i,j}(t_k))^2}{2\Delta_{i,j,k}^2} \right) \qquad (1)$$

with

$FP_{i,j}(t_k)$     the probability function for the $i^{th}$ aortic state and the $j^{th}$ set of ICG electrodes $5_{j,in}$, $5_{j,se}$ at the $k^{th}$ sensing time $t_k$,

exp     the exponential function, and

$\Delta_{i,j,k}$     an estimated uncertainty as to the sensing by the $j^{th}$ set $4_j$ of ICG electrodes $5_{j,in}$, $5_{j,se}$ and/or as to the reference impedance $f_{i,j}$ for the $i^{th}$ aortic state and the $j^{th}$ set $4_j$ of ICG electrodes $5_{j,in}$, $5_{j,se}$ at the $k^{th}$ sensing time $t_k$.

In alternative embodiments, the probability function may, e.g., be a log-normal distribution, a Pareto distribution, a gamma distribution, a Student's t-distribution, etc. with the respective maximum corresponding to the difference between the sensed and reference impedances being zero. In further alternative embodiments, correlations between the sensed impedances $Z_j$ may be considered, e.g., by extending the exponent of equation (1) with a covariance function depending on two or more sensed impedances $Z_j$.

In a further optional embodiment, which may be combined with the other embodiments, each reference impedance is an expansion of basis functions which depend on predefined aortic parameters of the aortic state. Therein, the product of probability functions also depends on the aortic parameters, i.e., $p(\mathbf{Z}|AS_i) = p(\mathbf{Z}|\mathbf{x}_i,AS_i)$ wherein $\mathbf{x}_i$ denotes the aortic parameters, and step 22 is performed by marginalising the product with respect to the aortic parameters.

The procedure of marginalising is known from statistics, in particular from Bayesian statistics, and is carried out by first multiplying the product (comparable to a likelihood in Bayesian statistics) with a predetermined prior probability for the aortic parameters given the aortic state and a predetermined prior probability for the aortic state, and then integrating the result over the aortic parameters. Thereby, each similarity value is computed in a statistical manner, also taking the aortic parameters into account. To this end, the values of the aortic parameters are determined when generating the surrogate models but do not have to be determined for the patient 2.

The aortic parameters are, in general, selected as parameters which allow for a distinction between the aortic states. For example, the aortic parameters of a healthy aortic state may be a haematocrit level and a maximum radius of the true lumen $R_{TL}$ (Fig. 5), i.e., $\mathbf{x}_1 = \{L_H, R_{TL}\}$, and/or the aortic parameters of a dissected aortic state may be a haematocrit level $L_H$, a maximum radius of the true lumen $R_{TL}$, a maximum radius of the false lumen $R_{FL}$ (Fig. 5), and a position of the false lumen relative to the true lumen (Fig. 5: $\alpha_{FL}$), i.e., $x_2 = \{L_H, R_{TL}, R_{FL}, \alpha_{FL}\}$.

In an optional variant, each similarity value is computed according to

$$p(AS_i | \mathbf{Z}) = \frac{p(AS_i)}{Vol(\mathbf{R}_i)} \int_{\mathbf{R}_i} p(\mathbf{Z} | \mathbf{x}_i, AS_i) dV_{\mathbf{x}_i} \qquad (2)$$

in particular according to

$$p(AS_i | \mathbf{Z}) = \frac{p(AS_i)}{Vol(\mathbf{R}_i)} \int_{\mathbf{R}_i} \prod_{\substack{j=1,\ldots,J \\ k=1,\ldots,K}} \frac{1}{\sqrt{2\pi \Delta_{i,j,k}^2}} \exp\left(-\frac{\left(Z_j(t_k) - f_{i,j}(\mathbf{x}_i, t_k)\right)^2}{2\Delta_{i,j,k}^2}\right) dV_{\mathbf{x}_i} \qquad (3)$$

with

$p(AS_i | \mathbf{Z})$     the similarity value of the $i^{th}$ aortic state $AS_i$ given the sensed impedances $\mathbf{Z}$,

$p(AS_i)$     a predetermined prior probability for the $i^{th}$ aortic state $AS_i$,

$\mathbf{R}_i$     a predetermined set of values of the aortic parameters $\mathbf{x}_i$ of the $i^{th}$ aortic state $AS_i$,

$Vol(\mathbf{R}_i)$     a hypervolume spanned by said predetermined set of values,

$dV_{\mathbf{x}_i}$     a hypervolume element,

$\int_{\mathbf{R}_i}$     an integration over the predetermined set of values, and

$f_{i,j}(\mathbf{x}_i, t_k)$     the reference impedance for the $i^{th}$ aortic state $AS_i$ and the $j^{th}$ set $4_j$ of ICG electrodes $5_{j,in}$, $5_{j,se}$ at the $k^{th}$ sensing time $t_k$ in dependence on the aortic parameters $\mathbf{x}_i$ of the $i^{th}$ aortic state $AS_i$.

The so computed similarity value is comparable to a posterior probability of the aortic state given the sensed impedances $Z_j$, and the product of probability functions is comparable to a (joint) likelihood (in the sense of Bayesian inference) which is marginalised via an uninformative uniform prior probability for the aortic parameters and multiplied by a predetermined prior probability for the aortic state. Thereby, the integration is restricted to the predetermined set of values of the respective aortic parameters. The set of values is a (often multidimensional) interval, for which the generated surrogate models of the respective aortic state yield meaningful results.

The afore-mentioned hypervolume is confined by the set of values $\mathbf{R}_i$ and is one-dimensional in case of a single aortic parameter, e.g., $\mathbf{x}_i = \{L_H\}$, two-dimensional in case of two aortic parameters, e.g., $\mathbf{x}_i = \{R_{TL}, R_{FL}\}$, and so forth. As an example, the maximum radius of the true lumen $R_{TL}$ may lie between 1 cm and 2.2 cm, preferably between 1.35 cm and 1.95 cm, and the maximum radius of the false lumen $R_{FL}$ between 0.1 cm and 2 cm, preferably between 0.3 cm and 1.5 cm for the healthy state, resulting in a set of values $\mathbf{R}_1 = \{[1.35\ \text{cm}; 1.95\ \text{cm}], [0.3\ \text{cm}; 1.5\ \text{cm}]\}$ for the healthy state and a hypervolume $Vol(\mathbf{R}_1) = (1.95 - 1.35)$ cm x $(1.5 - 0.3)$ cm $= 0.72$ cm$^2$. In other examples, the haematocrit level may lie between 35% and 55% and/or the position, given as an angle $\alpha_{FL}$ measured in a horizontal section of the thorax from a transverse axis A (Fig. 5), may lie between 2.9 and 3.65 rad.

Coming back to the description of the reference impedance by the surrogate model, when employing the aortic parameters, the basis functions are optionally time-independent multi-variate polynomials and the processor 8 calculates each reference impedance in step 22 from the surrogate model which describes the reference impedance by

$$f_{i,j}(\mathbf{x}_i, t_k) = f_{i,j}(\mathbf{x}_i; \mathbf{c}_i(t_k)) = \sum_{q=1,\ldots,Q} c_{i,j,q}(t_k) \cdot \phi_q(\mathbf{x}_i) \qquad (4)$$

with

$\phi_q(\mathbf{x}_i)$     a multi-variate polynomial in the aortic parameters $\mathbf{x}_i$ of the $i^{th}$ aortic state $AS_i$, wherein the polynomials are indexed by q,

$C_{i,j,q}(t_k)$     an expansion coefficient for the $i^{th}$ aortic state $AS_i$, the $j^{th}$ set of ICG electrodes $5_{j,in}$, $5_{j,se}$ and the $q^{th}$ polynomial $\phi_q(\mathbf{x}_i)$ at the $k^{th}$ sensing time $t_k$,

$\mathbf{c}_i$     the set of all expansion coefficients for the $i^{th}$ aortic state $AS_i$, and

$\sum_{q=1,\ldots,Q}$     a sum over all Q multi-variate polynomials $\phi_q(\mathbf{x}_i)$.

Optionally, the multi-variate polynomials may be chosen to be orthogonal with respect to a predetermined distribution of the aortic parameters, which yields a so-called polynomial chaos expansion.

Alternatively, other basis functions known in the art may be employed, e.g., non-polynomial basis functions such as Fourier basis functions, Gaussian basis function, etc., optionally combined with polynomial basis functions. In a further alternative embodiment the surrogate model comprises a neural network, e.g., a convolutional neural network, which describes the reference impedance and is trained on the aortic parameters as known in the art.

With reference to Figs. 4 and 5, it shall now be described how the surrogate models are generated. This can optionally be done prior to performing the method 9, e.g., on the basis of a large number of human individuals (or animals in case the patient 2 is animal) constituting said reference objects. Therein, the impedances of each individual are measured by said sets $4_j$ of ICG electrodes $5_{j,in}$, $5_{j,se}$ as described above for the patient 2 and the aortic state and all aortic parameters are also examined. Alternatively, the reference objects are finite element method (FEM) models of thoraces, an example of which is shown in Fig. 5 and marked with reference number 20; in this case, the generation of the surrogate models is either carried out prior to performing the method 9, i.e., separate from the measuring system 1 as mentioned above, or by the processor 8 in step 24, in either of the two variants by simulation.

Steps 25 - 28 shown in Fig. 4 illustrate details of the variant in which the processor 8 generates the surrogate model, i.e., the processor 8 carries out step 24. Steps 25 - 27 are repeated for each of the plurality of reference objects 20 having the same aortic state, and are repeated for each aortic state to generate all surrogate models. The values of the aortic parameters of the aortic state of the reference object 20 are determined in step 25. To achieve this, the processor 8 creates the FEM model 20 (Fig. 5) with predetermined values of the aortic parameters, e.g., to cover, with the entirety of reference objects, the abovementioned range of the aortic parameters in the simulation.

In step 26, the respective set $4_j$ of (here: simulated) ICG electrodes $5_{j,in}$, $5_{j,se}$ are applied (in simulation) at the respective spots $14_{j,in}$, $14_{j,se}$ on the FEM model 20 as shown in Fig. 5. As mentioned above, the spots $14_{j,in}$, $14_{j,se}$ on the thorax 15 of the patient 2 and on the FEM thorax 20 are equivalent.

In subsequent step 27, the impedance over time is quantified by means of the (simulated) applied ICG electrodes $5_{j,in}$, $5_{j,se}$. This is done by simulating the injection of the current I by means of the injector electrodes $5_{j,in}$ and the sensing of the impedance over time by means of the sensor electrodes $5_{j,se}$, e.g., by solving the Laplace equation for the electric potential in the FEM model 20 and considering the impact of blood flow through the aorta in the FEM model 20. Steps 26 and 27 are repeated for each set $4_j$ of ICG electrodes $5_{j,in}$, $5_{j,se}$ to obtain a number of quantified impedances for the reference object.

As illustrated in Fig. 5, the FEM model 20 can be of any desired level of detail, e.g., considering the reference object's lungs 29 and/or other organs, liquids, tissues, etc.

From the values of the aortic parameters determined in step 25 and the impedances over time quantified in step 27, the processor 8 then calculates the expansion coefficient of the respective surrogate model in step 28. Therefor, the processor 8 employs a regression or fitting method. Optionally, the processor 8 calculates the expansion coefficients of equation (4) according to

$$ c_{i,j,q}(t_k) = \sum_l \left( \left( \mathbf{U}_i^T \cdot \mathbf{U}_i \right)^{-1} \cdot \mathbf{U}_i^T \right)_{ql} Z_j^l(t_k) \qquad (5) $$

with

$\mathbf{U}_i = \phi_q(\mathbf{v}_i^l)$      a matrix for the $i^{th}$ aortic state $AS_i$ formed by evaluating the $q^{th}$ multi-variate polynomial at the determined values $\mathbf{v}_i^l$ of the aortic parameters $\mathbf{x}_i$ of the $l^{th}$ reference object having the $i^{th}$ aortic state $AS_i$, and

$Z_j^l(t_k)$      the quantified impedance of the $l^{th}$ reference object in the $j^{th}$ set of ICG electrodes $13_j$ at the $k^{th}$ sensing time $t_k$.

The calculated expansion coefficients, thus, parameterise the surrogate model such that the processor 8 can determine the reference impedances therefrom.

The present invention is not restricted to the specific embodiments and variants described in detail herein but encompasses all those variants, combinations and modifications thereof that fall within the scope of the appended claims.

**Claims**

1. A method for determining an aortic state of a patient (2) based on sensed impedances ($Z_1$, $Z_2$) by means of a processing device (6) which has a processor (8) and a memory (7) connected thereto, wherein each of said impedances ($Z_1$, $Z_2$) has been sensed over time by one of at least two different sets ($4_1$, $4_2$) of impedance cardiography, ICG, electrodes ($5_{1,in}$, $5_{1,se}$; $5_{2,in}$, $5_{2,se}$) connectable to the processing device (6) and configured to be placed in a tetra-polar electrode configuration at respective spots ($14_{1,in}$, $14_{1,se}$; $14_{2,in}$, $14_{2,se}$) on the patient's thorax (15), the method (11) comprising:

receiving (19) in said memory (7), from said sets ($4_1$, $4_2$) of ICG electrodes ($5_{1,in}$, $5_{1,se}$; $5_{2,in}$, $5_{2,se}$) said impedances ($Z_1$, $Z_2$);

prior to, during, or after said receiving (19), storing (21) in said memory (7), for each of a predetermined set of aortic states and each set ($4_1$, $4_2$) of ICG electrodes ($5_{1,in}$, $5_{1,se}$; $5_{2,in}$, $5_{2,se}$), a surrogate model generated for a plurality of reference objects (20) having that aortic state, which surrogate model describes a reference impedance over time;

computing (22), by means of the processor (8), for each aortic state of the set, a similarity value from a product of probability functions each of which depends on a difference between the sensed impedance ($Z_1$, $Z_2$) for a respective set ($4_1$, $4_2$) of ICG electrodes ($5_{1,in}$, $5_{1,se}$; $5_{2,in}$, $5_{2,se}$) and the reference impedance for that aortic state and the same set ($4_1$, $4_2$) of ICG electrodes ($5_{1,in}$, $5_{1,se}$; $5_{2,in}$, $5_{2,se}$); and

determining (23), by means of the processor (8), the aortic state with the highest computed similarity value as the patient's aortic state.

2. The method according to claim 1, wherein each probability function is a Gaussian function according to

$$\frac{1}{\sqrt{2\pi\Delta_{i,j,k}^2}}\exp\left(-\frac{\left(Z_j(t_k)-f_{i,j}(t_k)\right)^2}{2\Delta_{i,j,k}^2}\right)$$

with

exp the exponential function,

$Z_j(t_k)$ the sensed impedance for the $j^{th}$ set ($4_j$) of ICG electrodes ($5_{j,in}$, $5_{j,se}$) at the $k^{th}$ sensing time,

$f_{i,j}(t_k)$ the reference impedance for the $i^{th}$ aortic state and the $j^{th}$ set ($4_j$) of ICG electrodes ($5_{j,in}$, $5_{j,se}$) at the $k^{th}$ sensing time $t_k$, and

$\Delta_{i,j,k}$ an estimated uncertainty as to the sensing by the $j^{th}$ set ($4_j$) of ICG electrodes ($5_{j,in}$, $5_{j,se}$) and/or as to the reference impedance $f_{i,j}$ for the $i^{th}$ aortic state and the $j^{th}$ set ($4_j$) of ICG electrodes ($5_{j,in}$, $5_{j,se}$) at the $k^{th}$ sensing time $t_k$.

3. The method according to claim 1 or 2, wherein each reference impedance is an expansion of basis functions which depend on predefined aortic parameters ($R_{TL}$, $R_{FL}$, $\alpha_{FL}$) of the aortic state, and

wherein said step of computing (22) is performed by marginalising the product of probability functions with respect to the aortic parameters ($R_{TL}$, $R_{FL}$, $\alpha_{FL}$).

4. The method according to claim 3, wherein the aortic parameters of a healthy aortic state are a haematocrit level and a maximum radius ($R_{TL}$) of the true lumen.

5. The method according to claim 3 or 4, wherein the aortic parameters of a dissected aortic state are a haematocrit level, a maximum radius ($R_{TL}$) of the true lumen, a maximum radius ($R_{FL}$) of the false lumen, and a position ($\alpha_{FL}$) of the false lumen relative to the true lumen.

6. The method according to any one of claims 3 to 5, wherein, in said step of computing (22), each similarity value is computed according to

$$p\left(AS_i|\mathbf{Z}\right)=\frac{p(AS_i)}{Vol(\mathbf{R}_i)}\int_{\mathbf{R}_i} p\left(\mathbf{Z}|\mathbf{x}_i,AS_i\right)dV_{\mathbf{x}_i}$$

preferably according to

$$p\left(AS_i|\mathbf{Z}\right)=\frac{p(AS_i)}{Vol(\mathbf{R}_i)}\int_{\mathbf{R}_i}\prod_{\substack{j=1,\dots,J\\k=1,\dots,K}}\frac{1}{\sqrt{2\pi\Delta_{i,j,k}^2}}\exp\left(-\frac{\left(Z_j(t_k)-f_{i,j}(t_k,\mathbf{x}_i)\right)^2}{2\Delta_{i,j,k}^2}\right)dV_{\mathbf{x}_i}$$

with

p(AS$_i$|**Z**) the computed similarity value of the i$^{th}$ aortic state given the sensed impedances **Z,**
p(AS$_i$) a predetermined prior probability for the i$^{th}$ aortic state,
**R**$_i$ a predetermined set of values of the aortic parameters **x**$_i$ of the i$^{th}$ aortic state,
Vol(**R**$_i$) a hypervolume spanned by said predetermined set of values,
p(Z|**x**$_i$,AS$_i$) the product of probability functions,
$dV_{x_i}$ a hypervolume element,

$$\int_{\mathbf{R}_i}$$ an integration over the predetermined set of values,

$$\prod_{\substack{j=1,\ldots,J \\ k=1,\ldots,K}}$$ a product over all J sets of ICG electrodes and all K sensing times,

Z$_j$(t$_k$) the sensed impedance for the j$^{th}$ set (4$_1$, 4$_2$) of ICG electrodes (5$_{j,in}$, 5$_{j,se}$) at the k$^{th}$ sensing time t$_k$,
f$_{i,j}$(t$_k$) the reference impedance for the i$^{th}$ aortic state and the j$^{th}$ set (4$_j$) of ICG electrodes (5$_{j,in}$, 5$_{j,se}$) at the k$^{th}$ sensing time t$_k$, and
$\Delta_{i,j,k}$ an estimated uncertainty as to the sensing by the j$^{th}$ set (4$_j$) of ICG electrodes (5$_{j,in}$, 5$_{j,se}$) and/or as to the reference impedance f$_{i,j}$ for the i$^{th}$ aortic state and the j$^{th}$ set (4$_j$) of ICG electrodes (5$_{j,in}$, 5$_{j,se}$) at the k$^{th}$ sensing time t$_k$.

7. The method according to any one of claims 3 to 6, wherein, in said step of computing (22), each reference impedance is calculated by means of the processor (8) from the surrogate model which describes the reference impedance by

$$f_{i,j}(\mathbf{x}_i, t_k) = f_{i,j}(\mathbf{x}_i; \mathbf{c}_i(t_k)) = \sum_{q=1,\ldots,Q} c_{i,j,q}(t_k) \cdot \phi_q(\mathbf{x}_i)$$

with

f$_{i,j}$(**x**$_i$,t$_k$) the calculated reference impedance for the i$^{th}$ aortic state and the j$^{th}$ set (4$_j$) of ICG electrodes (5$_{j,in}$, 5$_{j,se}$) at the k$^{th}$ sensing time t$_k$ in dependence on the aortic parameters **x**$_i$ of the i$^{th}$ aortic state,
$\phi_q$(**x**$_i$) a multi-variate polynomial in the aortic parameters **x**$_i$ of the i$^{th}$ aortic state, wherein the polynomials are indexed by q,
c$_{i,j,q}$(t$_k$) an expansion coefficient for the i$^{th}$ aortic state, the j$^{th}$ set (4$_j$) of ICG electrodes (5$_{j,in}$, 5$_{j,se}$) and the q$^{th}$ polynomial at the k$^{th}$ sensing time,
**c**$_i$ the set of all expansion coefficients for the i$^{th}$ aortic state, and

$$\sum_{q=1,\ldots,Q}$$ a sum over all Q multi-variate polynomials.

8. The method according to any one of claims 3 to 7, wherein the reference objects are finite element method, FEM, models of thoraces (20) and the method (11) further comprises, prior to said step of storing (21), generating (24), by means of the processor (8), each surrogate model by:

determining (25), for each of the reference objects (20), the values of the aortic parameters (R$_{TL}$, R$_{FL}$, $\alpha_{FL}$) of the aortic state of that reference object (20);
applying (26), for each set (4$_1$, 4$_2$) of ICG electrodes (5$_{1,in}$, 5$_{1,se}$; 5$_{2,in}$, 5$_{2,se}$) and each of the reference objects (20), the ICG electrodes (5$_{1,in}$, 5$_{1,se}$; 5$_{2,in}$, 5$_{2,se}$) on that reference object's thorax at the respective spots (14$_{1,in}$, 14$_{1,se}$; 14$_{2,in}$, 14$_{2,se}$) and quantifying (27), by means of the applied ICG electrodes (5$_{1,in}$, 5$_{1,se}$; 5$_{2,in}$, 5$_{2,se}$), an impedance over time; and
calculating (28), by means of the processor (8) from the determined values of the aortic parameters and the quantified impedances, the expansion coefficients.

9. The method according to claim 8, wherein the expansion coefficients are calculated according to

$$c_{i,j,q}(t_k) = \sum_{l} \left( \left( \mathbf{U}_i^T \cdot \mathbf{U}_i \right)^{-1} \cdot \mathbf{U}_i^T \right)_{ql} Z_j^l(t_k)$$

with

$c_{i,j,q}(t_k)$ the expansion coefficient for the $i^{th}$ aortic state, the $j^{th}$ set ($4_j$) of ICG electrodes ($5_{j,in}$, $5_{j,se}$) and the $q^{th}$ polynomial at the $k^{th}$ sensing time $t_k$,

$\mathbf{U}_i = \phi_q(\mathbf{v}_i^l)$ a matrix for the $i^{th}$ aortic state formed by evaluating the $q^{th}$ multi-variate polynomial at the determined values $\mathbf{v}_i^l$ of the aortic parameters $\mathbf{x}_i$ of the $l^{th}$ reference object (20) having the $i^{th}$ aortic state, and

$Z_j^l(t_k)$ the quantified impedance of the $l^{th}$ reference object (20) in the $j^{th}$ set ($4_j$) of ICG electrodes ($5_{j,in}$, $5_{j,se}$) at the $k^{th}$ sensing time $t_k$.

10. A processing device for determining an aortic state of a patient (2), the processing device (6) having

a memory (7) which is configured to receive (19), from at least two different sets ($4_1$, $4_2$) of impedance cardiography, ICG, electrodes ($5_{1,in}$, $5_{1,se}$; $5_{2,in}$, $5_{2,se}$) connectable to said processing device (6) and configured to be placed in a tetra-polar electrode configuration at respective spots ($14_{1,in}$, $14_{1,se}$; $14_{2,in}$, $14_{2,se}$) on the patient's thorax (15), an impedance ($Z_1$, $Z_2$) sensed over time by that set ($4_1$, $4_2$) of ICG electrodes ($5_{1,in}$, $5_{1,se}$; $5_{2,in}$, $5_{2,se}$), and to store (21), for each of a predetermined set of aortic states and each set ($4_1$, $4_2$) of ICG electrodes ($5_{1,in}$, $5_{1,se}$; $5_{2,in}$, $5_{2,se}$), a surrogate model generated for a plurality of reference objects (20) having that aortic state, which surrogate model describes a reference impedance over time; and

a processor (8) connected to said memory (7) and configured to compute (22), for each aortic state of the set ($4_1$, $4_2$), a similarity value from a product of probability functions each of which depends on a difference between the sensed impedance ($Z_1$, $Z_2$) for a respective set ($4_1$, $4_2$) of ICG electrodes ($5_{1,in}$, $5_{1,se}$; $5_{2,in}$, $5_{2,se}$) and the reference impedance for that aortic state and the same set ($4_1$, $4_2$) of ICG electrodes ($5_{1,in}$, $5_{1,se}$; $5_{2,in}$, $5_{2,se}$), and to determine (23) the aortic state with the highest computed similarity value as the patient's aortic state.

11. The processing device according to claim 10, wherein each reference impedance is an expansion of basis functions which depend on predefined aortic parameters ($R_{TL}$, $R_{FL}$, $\alpha_{FL}$) of the aortic state, and

wherein the processor (8) is configured to perform said step of computing (22) by marginalising the product of probability functions with respect to the aortic parameters ($R_{TL}$, $R_{FL}$, $\alpha_{FL}$).

12. The processing device according to claim 11, wherein the processor (8) is configured to compute the similarity value of each aortic state according to

$$p\left(AS_i | \mathbf{Z}\right) = \frac{p(AS_i)}{Vol(\mathbf{R}_i)} \int_{\mathbf{R}_i} p\left(\mathbf{Z} | \mathbf{x}_i, AS_i\right) dV_{\mathbf{x}_i}$$

preferably according to

$$p\left(AS_i | \mathbf{Z}\right) = \frac{p(AS_i)}{Vol(\mathbf{R}_i)} \int_{\mathbf{R}_i} \prod_{\substack{j=1,\ldots,J \\ k=1,\ldots,K}} \frac{1}{\sqrt{2\pi\Delta_{i,j,k}^2}} \exp\left(-\frac{\left(Z_j(t_k) - f_{i,j}(\mathbf{x}_i, t_k)\right)^2}{2\Delta_{i,j,k}^2}\right) dV_{\mathbf{x}_i}$$

with

$p(AS_i | \mathbf{Z})$ the computed similarity value of the $i^{th}$ aortic state given the sensed impedances $\mathbf{Z}$,

$p(AS_i)$ a predetermined prior probability for the $i^{th}$ aortic state $AS_i$,

$\mathbf{R}_i$ a predetermined set of values of the aortic parameters $\mathbf{x}_i$ of the $i^{th}$ aortic state,

$Vol(\mathbf{R}_i)$ a hypervolume spanned by said predetermined set of values,

$p(\mathbf{Z} | \mathbf{x}_i, AS_i)$ the product of probability functions,

$dV_{\mathbf{x}_i}$ a hypervolume element,

$\int_{\mathbf{R}_i}$ an integration over the predetermined set of values, a product over all J sets of ICG electrodes and all K sensing times,

$Z_j(t_k)$ the sensed impedance for the $j^{th}$ set ($4_j$) of ICG electrodes ($5_{j,in}$, $5_{j,se}$) at the $k^{th}$ sensing time $t_k$,

$f_{i,j}(\mathbf{x}_i, t_k)$ the reference impedance for the $i^{th}$ aortic state and the $j^{th}$ set ($4_j$) of ICG electrodes ($5_{j,in}$, $5_{j,se}$) at the $k^{th}$

sensing time $t_k$ in dependence on the aortic parameters $\mathbf{x}_i$ of the $i^{th}$ aortic state, and

$\Delta_{i,j,k}$ an estimated uncertainty as to the sensing by the $j^{th}$ set $(4_j)$ of ICG electrodes $(5_{j,in}, 5_{j,se})$ and/or as to the reference impedance $f_{i,j}$ for the $i^{th}$ aortic state and the $j^{th}$ set $(4_j)$ of ICG electrodes $(5_{j,in}, 5_{j,se})$ at the $k^{th}$ sensing time $t_k$.

13. The processing device according to claim 11 or 12, wherein the processor (8) is further configured to calculate each reference impedance from the surrogate model which describes the reference impedance by

$$f_{i,j}(\mathbf{x}_i, t_k) = f_{i,j}(\mathbf{x}_i; \mathbf{c}_i(t_k)) = \sum_{q=1,\dots,Q} c_{i,j,q}(t_k) \cdot \phi_q(\mathbf{x}_i)$$

with

$f_{i,j}(\mathbf{x}_{i}, t_k)$ the reference impedance for the $i^{th}$ aortic state and the $j^{th}$ set $(4_j)$ of ICG electrodes $(5_{j,in}, 5_{j,se})$ at the $k^{th}$ sensing time $t_k$ in dependence on the aortic parameters $\mathbf{x}_i$ of the $i^{th}$ aortic state,

$\phi_q(\mathbf{x}_i)$ a multi-variate polynomial in the aortic parameters $\mathbf{x}_i$ of the $i^{th}$ aortic state, wherein the polynomials are indexed by q,

$c_{i,j,q}(t_k)$ an expansion coefficient for the $i^{th}$ aortic state, the $j^{th}$ set $(4_j)$ of ICG electrodes $(5_{j,in}, 5_{j,se})$ and the $q^{th}$ polynomial at the $k^{th}$ sensing time $t_k$,

$\mathbf{c}_i$ the set of all expansion coefficients for the $i^{th}$ aortic state, and

$\sum_{q=1,\dots,Q}$ a sum over all Q multi-variate polynomials.

14. A measuring system comprising the processing device (6) according to any one of claims 10 to 13 and said at least two different sets $(4_1, 4_2)$ of ICG electrodes $(5_{1,in}, 5_{1,se}; 5_{2,in}, 5_{2,se})$, wherein each set $(4_1, 4_2)$ of ICG electrodes $(5_{1,in}, 5_{1,se}; 5_{2,in}, 5_{2,se})$ is configured to be placed (16) in the tetra-polar electrode configuration at the respective spots $(14_{1,in}, 14_{1,se}; 14_{2,in}, 14_{2,se})$ on the patient's thorax (15), to sense (17) said impedance $(Z_1, Z_2)$ over time and to transmit (18) the sensed impedance $(Z_1, Z_2)$ to the memory (7).

**Patentansprüche**

1. Verfahren zum Ermitteln eines Aortenzustands eines Patienten (2) basierend auf erfassten Impedanzen $(Z_1, Z_2)$ mithilfe einer Verarbeitungsvorrichtung (6), die einen Prozessor (8) und einen damit verbundenen Speicher (7) hat, wobei jede der genannten Impedanzen $(Z_1, Z_2)$ über die Zeit von einem von zumindest zwei verschiedenen Sätzen $(4_1, 4_2)$ von Impedanzkardiographie-, IKG-, Elektroden $(5_{1,in}, 5_{1,se}; 5_{2,in}, 5_{2,se})$ erfasst wurde, die mit der Verarbeitungsvorrichtung (6) verbindbar und dazu ausgebildet sind, in einer tetrapolaren Elektrodenkonfiguration an jeweiligen Stellen $(14_{1,in}, 14_{1,se}; 14_{2,in}, 14_{2,se})$ am Thorax (15) des Patienten platziert zu werden, das Verfahren (11) umfassend:

Empfangen (19) im genannten Speicher (7) der genannten Impedanzen $(Z_1, Z_2)$ von den genannten Sätzen $(4_1, 4_2)$ von IKG-Elektroden $(5_{1,in}, 5_{1,se}; 5_{2,in}, 5_{2,se})$;

vor, während oder nach dem genannten Empfangen (19), Speichern (21) im genannten Speicher (7), für jeden aus einem vorgegebenen Satz von Aortenzuständen und jeden Satz $(4_1, 4_2)$ von IKG-Elektroden $(5_{1,in}, 5_{1,se}; 5_{2,in}, 5_{2,se})$, eines Surrogatmodells, das für eine Vielzahl von Referenzobjekten (20) mit diesem Aortenzustand erzeugt wurde, wobei das Surrogatmodell eine Referenzimpedanz über die Zeit beschreibt;

Berechnen (22), mithilfe des Prozessors (8), für jeden Aortenzustand des Satzes, eines Ähnlichkeitswerts aus einem Produkt von Wahrscheinlichkeitsfunktionen, von denen jede von einer Differenz zwischen der erfassten Impedanz $(Z_1, Z_2)$ für einen jeweiligen Satz $(4_1, 4_2)$ von IKG-Elektroden $(5_{1,in}, 5_{1,se}; 5_{2,in}, 5_{2,se})$ und der Referenzimpedanz für diesen Aortenzustand und denselben Satz $(4_1, 4_2)$ von IKG-Elektroden $(5_{1,in}, 5_{1,se}; 5_{2,in}, 5_{2,se})$ abhängt; und

Ermitteln (23), mithilfe des Prozessors (8), des Aortenzustands mit dem höchsten berechneten Ähnlichkeitswert als den Aortenzustand des Patienten.

2. Verfahren nach Anspruch 1, wobei jede Wahrscheinlichkeitsfunktion eine Gauß'sche Funktion gemäß

$$\frac{1}{\sqrt{2\pi\Delta_{i,j,k}^2}}\exp\left(-\frac{\left(Z_j(t_k)-f_{i,j}(t_k)\right)^2}{2\Delta_{i,j,k}^2}\right)$$

ist, mit

exp der Exponentialfunktion,

$Z_j(t_k)$ der erfassten Impedanz für den j-ten Satz ($4_j$) von IKG-Elektroden ($5_{j,in}$, $5_{j,se}$) zum k-ten Erfassungszeitpunkt,

$f_{i,j}(t_k)$ der Referenzimpedanz für den i-ten Aortenzustand und den j-ten Satz ($4_j$) von IKG-Elektroden ($5_{j,in}$, $5_{j,se}$) zum k-ten Erfassungszeitpunkt $t_k$, und

$\Delta_{i,j,k}$ einer geschätzten Unsicherheit hinsichtlich der Erfassung durch den j-ten Satz ($4_j$) von IKG-Elektroden ($5_{j,in}$, $5_{j,se}$) und/oder hinsichtlich der Referenzimpedanz $f_{i,j}$ für den i-ten Aortenzustand und den j-ten Satz ($4_j$) von IKG-Elektroden ($5_{j,in}$, $5_{j,se}$) zum k-ten Erfassungszeitpunkt $t_k$.

3. Verfahren nach Anspruch 1 oder 2, wobei jede Referenzimpedanz eine Entwicklung von Basisfunktionen ist, die von vordefinierten Aortenparametern ($R_{TL}$, $R_{FL}$, $\alpha_{FL}$) des Aortenzustands abhängen, und
wobei der genannte Schritt des Berechnens (22) durch Marginalisieren des Produkts von Wahrscheinlichkeitsfunktionen in Bezug auf die Aortenparameter ($R_{TL}$, $R_{FL}$, $\alpha_{FL}$) durchgeführt wird.

4. Verfahren nach Anspruch 3, wobei die Aortenparameter eines gesunden Aortenzustands ein Hämatokritniveau und ein maximaler Radius ($R_{TL}$) des wahren Lumens sind.

5. Verfahren nach Anspruch 3 oder 4, wobei die Aortenparameter eines dissektionierten Aortenzustands ein Hämatokritniveau, ein maximaler Radius ($R_{TL}$) des wahren Lumens, ein maximaler Radius ($R_{FL}$) des falschen Lumens und eine Position ($\alpha_{FL}$) des falschen Lumens relativ zum wahren Lumen sind.

6. Verfahren nach einem der Ansprüche 3 bis 5, wobei im genannten Schritt des Berechnens (22) jeder Ähnlichkeitswert gemäß

$$p(AS_i|\mathbf{Z})=\frac{p(AS_i)}{Vol(\mathbf{R}_i)}\int_{\mathbf{R}_i}p(\mathbf{Z}|\mathbf{x}_i,AS_i)dV_{\mathbf{x}_i}$$

bevorzugt gemäß

$$p(AS_i|\mathbf{Z})=\frac{p(AS_i)}{Vol(\mathbf{R}_i)}\int_{\mathbf{R}_i}\prod_{\substack{j=1,\dots,J\\k=1,\dots,K}}\frac{1}{\sqrt{2\pi\Delta_{i,j,k}^2}}\exp\left(-\frac{\left(Z_j(t_k)-f_{i,j}(t_k,\mathbf{x}_i)\right)^2}{2\Delta_{i,j,k}^2}\right)dV_{\mathbf{x}_i}$$

berechnet wird, mit

$p(AS_i|\mathbf{Z})$ dem berechneten Ähnlichkeitswert des i-ten Aortenzustands unter Berücksichtigung der erfassten Impedanzen $\mathbf{Z}$,

$p(AS_i)$ einer vorgegebenen a-priori-Wahrscheinlichkeit für den i-ten Aortenzustand,

$\mathbf{R}_i$ einem vorgegebenen Satz von Werten der Aortenparameter $\mathbf{x}_i$ des i-ten Aortenzustands,

$Vol(\mathbf{R}_i)$ einem von dem genannten vorgegebenen Satz von Werten aufgespannten Hypervolumen,

$p(\mathbf{Z}|\mathbf{x}_i,AS_i)$ dem Produkt von Wahrscheinlichkeitsfunktionen,

$dV_{\mathbf{x}_i}$ einem Hypervolumenelement,

$\int_{\mathbf{R}_i}$ einer Integration über den vorgegebenen Satz von Werten,

$\prod_{\substack{j=1,\dots,J\\k=1,\dots,K}}$ einem Produkt über alle J Sätze von IKG-Elektroden und alle K Erfassungszeiten,

$Z_j(t_k)$ der erfassten Impedanz für den j-ten Satz ($4_1$, $4_2$) von IKG-Elektroden ($5_{j,in}$, $5_{j,se}$) zum k-ten Erfassungszeitpunkt $t_k$,

$f_{i,j}(t_k)$ der Referenzimpedanz für den i-ten Aortenzustand und den j-ten Satz ($4_j$) von IKG-Elektroden ($5_{j,in}$, $5_{j,se}$) zum k-ten Erfassungszeitpunkt $t_k$, und

$\Delta_{i,j,k}$ einer geschätzten Unsicherheit hinsichtlich der Erfassung durch den j-ten Satz ($4_j$) von IKG-Elektroden ($5_{j,in}$, $5_{j,se}$) und/oder hinsichtlich der Referenzimpedanz $f_{i,j}$ für den i-ten Aortenzustand und den j-ten Satz ($4_j$) von IKG-Elektroden ($5_{j,in}$, $5_{j,se}$) zum k-ten Erfassungszeitpunkt $t_k$.

7. Verfahren nach einem der Ansprüche 3 bis 6, wobei im genannten Schritt des Berechnens (22) jede Referenzimpedanz mithilfe des Prozessors (8) aus dem Surrogatmodell errechnet wird, welches die Referenzimpedanz durch

$$f_{i,j}(\mathbf{x}_i, t_k) = f_{i,j}(\mathbf{x}_i; \mathbf{c}_i(t_k)) = \sum_{q=1,\ldots,Q} c_{i,j,q}(t_k) \cdot \phi_q(\mathbf{x}_i)$$

beschreibt, mit

$f_{i,j}(\mathbf{x}_i, t_k)$ der errechneten Referenzimpedanz für den i-ten Aortenzustand und den j-ten Satz ($4_j$) von IKG-Elektroden ($5_{j,in}$, $5_{j,se}$) zum k-ten Erfassungszeitpunkt $t_k$ in Abhängigkeit von den Aortenparametern $\mathbf{x}_i$ des i-ten Aortenzustands,

$\phi_q(\mathbf{x}_i)$ einem multivariaten Polynom in den Aortenparametern $\mathbf{x}_i$ des i-ten Aortenzustands, wobei die Polynome durch q indexiert sind,

$c_{i,j,q}(t_k)$ einem Entwicklungskoeffizienten für den i-ten Aortenzustand, den j-ten Satz ($4_j$) von IKG-Elektroden ($5_{j,in}$, $5_{j,se}$) und das q-te Polynom zum k-ten Erfassungszeitpunkt,

$\mathbf{c}_i$ dem Satz aller Entwicklungskoeffizienten für den i-ten Aortenzustand, und

$\displaystyle\sum_{q=1,\ldots,Q}$ eine Summe über alle Q multivariaten Polynome.

8. Verfahren nach einem der Ansprüche 3 bis 7, wobei die Referenzobjekte Finite-Elemente-Method-, FEM-, Modelle von Thoraxen (20) sind und das Verfahren (11) ferner vor dem genannten Schritt des Speicherns (21) Erzeugen (24) jedes Surrogatmodells mithilfe des Prozessors (8) umfasst, durch:

Ermitteln (25), für jedes der Referenzobjekte (20), der Werte der Aortenparameter ($R_{TL}$, $R_{FL}$, $\alpha_{FL}$) des Aortenzustands dieses Referenzobjekts (20);

Anbringen (26), für jeden Satz ($4_1$, $4_2$) von IKG-Elektroden ($5_{1,in}$, $5_{1,se}$; $5_{2,in}$, $5_{2,se}$) und jedes der Referenzobjekte (20), der IKG-Elektroden ($5_{1,in}$, $5_{1,se}$; $5_{2,in}$, $5_{2,se}$) auf dem Thorax dieses Referenzobjekts an den jeweiligen Stellen ($14_{1,in}$, $14_{1,se}$; $14_{2,in}$, $14_{2,se}$) und Quantifizieren (27), mithilfe der angebrachten IKG-Elektroden ($5_{1,in}$, $5_{1,se}$; $5_{2,in}$, $5_{2,se}$), einer Impedanz über die Zeit; und

Errechnen (28) der Entwicklungskoeffizienten mithilfe des Prozessors (8) aus den ermittelten Werten der Aortenparameter und den quantifizierten Impedanzen.

9. Verfahren nach Anspruch 8, wobei die Entwicklungskoeffizienten gemäß

$$c_{i,j,q}(t_k) = \sum_l \left(\left(\mathbf{U}_i^T \cdot \mathbf{U}_i\right)^{-1} \cdot \mathbf{U}_i^T\right)_{ql} Z_j^l(t_k)$$

errechnet werden, mit

$c_{i,j,q}(t_k)$ dem Entwicklungskoeffizienten für den i-ten Aortenzustand, dem j-ten Satz ($4_j$) von IKG-Elektroden ($5_{j,in}$, $5_{j,se}$) und dem q-ten Polynom zum k-ten Erfassungszeitpunkt $t_k$,

$\mathbf{U}_i = \phi_q(\mathbf{v}_i^l)$ einer Matrix für den i-ten Aortenzustand, gebildet durch Auswerten des q-ten multivariaten Polynoms an den ermittelten Werten $\mathbf{v}_i^l$ der Aortenparameter $\mathbf{x}_i$ des l-ten Referenzobjekts (20), welches den i-ten Aortenzustand hat, und

$Z_j^l(t_k)$ der quantifizierten Impedanz des l-ten Referenzobjekts (20) im j-ten Satz ($4_j$) von IKG-Elektroden ($5_{j,in}$, $5_{j,se}$) zum k-ten Erfassungszeitpunkt $t_k$.

10. Verarbeitungsvorrichtung zum Ermitteln eines Aortenzustands eines Patienten (2), wobei die Verarbeitungsvorrichtung (6)

einen Speicher (7), der dazu ausgebildet ist, von zumindest zwei verschiedenen Sätzen ($4_1$, $4_2$) von Impe- danzkardiographie-, IKG-, Elektroden ($5_{1,in}$, $5_{1,se}$; $5_{2,in}$, $5_{2,se}$), die mit der genannten Verarbeitungsvorrichtung (6) verbindbar und dazu ausgebildet sind, in einer tetrapolaren Elektrodenkonfiguration an jeweiligen Stellen ($14_{1,in}$, $14_{1,se}$; $14_{2,in}$, $14_{2,se}$) am Thorax (15) des Patienten platziert zu werden, eine Impedanz ($Z_1$, $Z_2$) zu empfangen (19), die über die Zeit von diesem Satz ($4_1$, $4_2$) von IKG-Elektroden ($5_{1,in}$, $5_{1,se}$; $5_{2,in}$, $5_{2,se}$) erfasst wird, und für jeden aus einem vorgegebenen Satz von Aortenzuständen und jeden Satz ($4_1$, $4_2$) von IKG- Elektroden ($5_{1,in}$, $5_{1,se}$; $5_{2,in}$, $5_{2,se}$) ein Surrogatmodell zu speichern (21), das für eine Vielzahl von Referenzobjekten (20) mit diesem Aortenzustand erzeugt wurde, wobei das Surrogatmodell eine Referenzimpedanz über die Zeit beschreibt; und
einen Prozessor (8) hat, der mit dem genannten Speicher (7) verbunden und dazu ausgebildet ist, für jeden Aortenzustand des Satzes ($4_1$, $4_2$) einen Ähnlichkeitswert aus einem Produkt von Wahrscheinlichkeitsfunktionen zu berechnen (22), von denen jede von einer Differenz zwischen der erfassten Impedanz ($Z_1$, $Z_2$) für einen jeweiligen Satz ($4_1$, $4_2$) von IKG-Elektroden ($5_{1,in}$, $5_{1,se}$; $5_{2,in}$, $5_{2,se}$) und der Referenzimpedanz für diesen Aortenzustand und denselben Satz ($4_1$, $4_2$) von IKG-Elektroden ($5_{1,in}$, $5_{1,se}$; $5_{2,in}$, $5_{2,se}$) abhängt, und den Aortenzustand mit dem höchsten berechneten Ähnlichkeitswert als den Aortenzustand des Patienten zu ermitteln (23).

11. Verarbeitungsvorrichtung nach Anspruch 10, wobei jede Referenzimpedanz eine Entwicklung von Basisfunktionen ist, die von vordefinierten Aortenparametern ($R_{TL}$, $R_{FL}$, $\alpha_{FL}$) des Aortenzustands abhängen, und
wobei der Prozessor (8) dazu ausgebildet ist, den genannten Schritt des Berechnens (22) durch Marginalisieren des Produkts von Wahrscheinlichkeitsfunktionen in Bezug auf die Aortenparameter ($R_{TL}$, $R_{FL}$, $\alpha_{FL}$) durchzuführen.

12. Verarbeitungsvorrichtung nach Anspruch 11, wobei der Prozessor (8) dazu ausgebildet ist, den Ähnlichkeitswert jedes Aortenzustands gemäß

$$p\left(AS_i\,|\,\mathbf{Z}\right)=\frac{p\left(AS_i\right)}{Vol\left(\mathbf{R}_i\right)}\int_{\mathbf{R}_i}p\left(\mathbf{Z}\,|\,\mathbf{x}_i,AS_i\right)dV_{\mathbf{x}_i}$$

bevorzugt gemäß

$$p\left(AS_i\,|\,\mathbf{Z}\right)=\frac{p\left(AS_i\right)}{Vol\left(\mathbf{R}_i\right)}\int_{\mathbf{R}_i}\prod_{\substack{j=1,...,J\\k=1,...,K}}\frac{1}{\sqrt{2\pi\Delta^2_{i,j,k}}}\exp\left(-\frac{\left(Z_j(t_k)-f_{i,j}(\mathbf{x}_i,t_k)\right)^2}{2\Delta^2_{i,j,k}}\right)dV_{\mathbf{x}_i}$$

zu berechnen, mit

$p(AS_i|\mathbf{Z})$ dem berechneten Ähnlichkeitswert des i-ten Aortenzustands unter Berücksichtigung der erfassten Impedanzen $\mathbf{Z}$,
$p(AS_i)$ einer vorgegebenen a-priori-Wahrscheinlichkeit für den i-ten Aortenzustand $AS_i$,
$\mathbf{R}_i$ einem vorgegebenen Satz von Werten der Aortenparameter $\mathbf{x}_i$ des i-ten Aortenzustands,
$Vol(\mathbf{R}_i)$ einem von dem genannten vorgegebenen Satz von Werten aufgespannten Hypervolumen,
$p(\mathbf{Z}|\mathbf{x}_i,AS_i)$ dem Produkt von Wahrscheinlichkeitsfunktionen,
$dV_{\mathbf{x}_i}$ einem Hypervolumenelement,

$\int_{\mathbf{R}_i}$ einer Integration über den vorgegebenen Satz von Werten,

$\prod_{\substack{j=1,...,J\\k=1,...,K}}$ einem Produkt über alle J Sätze von IKG-Elektroden und alle K Erfassungszeiten,

$Z_j(t_k)$ der erfassten Impedanz für den j-ten Satz ($4_j$) von IKG-Elektroden ($5_{j,in}$, $5_{j,se}$) zum k-ten Erfassungszeitpunkt $t_k$,
$f_{i,j}(\mathbf{x}_i,t_k)$ der Referenzimpedanz für den i-ten Aortenzustand und den j-ten Satz ($4_j$) von IKG-Elektroden ($5_{j,in}$, $5_{j,se}$)

zum k-ten Erfassungszeitpunkt $t_k$ in Abhängigkeit von den Aortenparametern $\mathbf{x}_i$ des i-ten Aortenzustands, und $\Delta_{i,j,k}$ einer geschätzten Unsicherheit hinsichtlich der Erfassung durch den j-ten Satz ($4_j$) von IKG-Elektroden ($5_{j,in}$, $5_{j,se}$) und/oder hinsichtlich der Referenzimpedanz $f_{i,j}$ für den i-ten Aortenzustand und den j-ten Satz ($4_j$) von IKG-Elektroden ($5_{j,in}$, $5_{j,se}$) zum k-ten Erfassungszeitpunkt $t_k$.

**13.** Verarbeitungsvorrichtung nach Anspruch 11 oder 12, wobei der Prozessor (8) ferner dazu ausgebildet ist, jede Referenzimpedanz aus dem Surrogatmodell zu errechnen, welches die Referenzimpedanz durch

$$f_{i,j}\left(\mathbf{x}_i, t_k\right) = f_{i,j}\left(\mathbf{x}_i; \mathbf{c}_i\left(t_k\right)\right) = \sum_{q=1,\ldots,Q} c_{i,j,q}\left(t_k\right) \cdot \phi_q\left(\mathbf{x}_i\right)$$

beschreibt, mit

$f_{i,j}(\mathbf{x}_i, t_k)$ der Referenzimpedanz für den i-ten Aortenzustand und den j-ten Satz ($4_j$) von IKG-Elektroden ($5_{j,in}$, $5_{j,se}$) zum k-ten Erfassungszeitpunkt $t_k$ in Abhängigkeit von den Aortenparametern $\mathbf{x}_i$ des i-ten Aortenzustands,
$\phi_q(\mathbf{x}_i)$ einem multivariaten Polynom in den Aortenparametern $\mathbf{x}_i$ des i-ten Aortenzustands, wobei die Polynome durch q indexiert sind,
$c_{i,j,q}(t_k)$ einem Entwicklungskoeffizienten für den i-ten Aortenzustand, den j-ten Satz ($4_j$) von IKG-Elektroden ($5_{j,in}$, $5_{j,se}$) und das q-te Polynom zum k-ten Erfassungszeitpunkt $t_k$,
$\mathbf{c}_i$ dem Satz aller Entwicklungskoeffizienten für den i-ten Aortenzustand, und

$\sum_{q=1,\ldots,Q}$ eine Summe über alle Q multivariaten Polynome.

**14.** Messsystem, umfassend die Verarbeitungsvorrichtung (6) nach einem der Ansprüche 10 bis 13 und die genannten zumindest zwei verschiedenen Sätze ($4_1$, $4_2$) von IKG-Elektroden ($5_{1,in}$, $5_{1,se}$; $5_{2,in}$, $5_{2,se}$), wobei jeder Satz ($4_1$, $4_2$) von IKG-Elektroden ($5_{1,in}$, $5_{1,se}$; $5_{2,in}$, $5_{2,se}$) dazu ausgebildet ist, in der tetrapolaren Elektrodenkonfiguration an den jeweiligen Stellen ($14_{1,in}$, $14_{1,se}$; $14_{2,in}$, $14_{2,se}$) am Thorax (15) des Patienten platziert (16) zu werden, um die genannte Impedanz ($Z_1$, $Z_2$) über die Zeit zu erfassen (17) und die erfasste Impedanz ($Z_1$, $Z_2$) an den Speicher (7) zu übertragen (18).

**Revendications**

**1.** Méthode pour la détermination d'un état aortique d'un patient (2) en se basant sur les impédances ($Z_1$, $Z_2$) détectées au moyen d'un dispositif de traitement (6) qui a un processeur (8) et une mémoire (7) connectée à celui-ci, où chacune desdites impédances ($Z_1$, $Z_2$) a été détectée au cours du temps par un parmi au moins deux ensembles ($4_1$, $4_2$) différents d'électrodes de cardiographie d'impédance, ICG, ($5_{1,in}$, $5_{1,se}$; $5_{2,in}$, $5_{2,se}$) pouvant être connectées au dispositif de traitement (6) et conçues pour être placées dans une configuration d'électrodes tétrapolaire à des emplacements ($14_{1,in}$, $14_{1,se}$; $14_{2,in}$, $14_{2,se}$) respectifs sur le thorax du patient (15), la méthode (11) comprenant :

la réception (19) dans ladite mémoire (7), depuis lesdits ensembles ($4_1$, $4_2$) d'électrodes d'ICG ($5_{1,in}$, $5_{1,se}$; $5_{2,in}$, $5_{2,se}$), desdites impédances ($Z_1$, $Z_2$) ;
avant, pendant ou après ladite étape de réception (19), le stockage (21) dans ladite mémoire (7), pour chacun parmi un ensemble prédéterminé d'états aortiques et chaque ensemble ($4_1$, $4_2$) d'électrodes d'ICG ($5_{1,in}$, $5_{1,se}$; $5_{2,in}$, $5_{2,se}$), d'un modèle de substitution généré pour une pluralité d'objets de référence (20) ayant cet état aortique, lequel modèle de substitution décrit une impédance de référence au cours du temps ;
le calcul (22), au moyen du processeur (8), pour chaque état aortique de l'ensemble, d'une valeur de similitude depuis un produit de fonction de probabilité, chacune dépendant d'une différence entre l'impédance ($Z_1$, $Z_2$) détectée pour un ensemble ($4_1$, $4_2$) respectif d'électrodes d'ICG ($5_{1,in}$, $5_{1,se}$; $5_{2,in}$, $5_{2,se}$) et l'impédance de référence pour cet état aortique et le même ensemble ($4_1$, $4_2$) d'électrodes d'ICG ($5_{1,in}$, $5_{1,se}$; $5_{2,in}$, $5_{2,se}$) ; et
la détermination (23), au moyen du processeur (8), de l'état aortique avec la valeur de similitude calculée la plus élevée en tant qu'état aortique du patient.

**2.** Méthode selon la revendication 1, dans laquelle chaque fonction de probabilité est une fonction gaussienne selon

$$\frac{1}{\sqrt{2\pi\Delta_{i,j,k}^2}}\exp\left(-\frac{\left(Z_j(t_k)-f_{i,j}(t_k)\right)^2}{2\Delta_{i,j,k}^2}\right)$$

avec

exp la fonction exponentielle,

$Z_j(t_k)$ l'impédance détectée pour le $j^e$ ensemble ($4_j$) d'électrodes d'ICG ($5_{j,in}$, $5_{j,se}$) au $k^e$ temps de détection,

$f_{i,j}(t_k)$ l'impédance de référence pour le $i^e$ état aortique et le $j^e$ ensemble ($4_j$) d'électrodes d'ICG ($5_{j,in}$, $5_{j,se}$) au $k^e$ temps de détection $t_k$, et

$\Delta_{i,j,k}$ une incertitude estimée quant à la détection par le $j^e$ ensemble ($4_j$) d'électrodes d'ICG ($5_{j,in}$, $5_{j,se}$) et/ou quant à l'impédance de référence $f_{i,j}$ pour le $i^e$ état aortique et le $j^e$ ensemble ($4_j$) d'électrodes d'ICG ($5_{j,in}$, $5_{j,se}$) au $k^e$ temps de détection $t_k$.

3. Méthode selon la revendication 1 ou 2, dans laquelle chaque impédance de référence est un développement des fonctions de base qui dépendent de paramètres aortiques prédéfinis ($R_{TL}$, $R_{FL}$, $\alpha_{FL}$) de l'état aortique, et dans laquelle ladite étape de calcul (22) est réalisée par la marginalisation du produit de fonctions de probabilité par rapport aux paramètres aortiques ($R_{TL}$, $R_{FL}$, $\alpha_{FL}$).

4. Méthode selon la revendication 3, dans laquelle les paramètres aortiques d'un état aortique sain sont un taux d'hématocrite et un rayon maximal ($R_{TL}$) de la vraie lumière.

5. Méthode selon la revendication 3 ou 4, dans laquelle les paramètres aortiques d'un état aortique disséqué sont un taux d'hématocrite, un rayon maximal ($R_{TL}$) de la vraie lumière, un rayon maximal ($R_{FL}$) de la fausse lumière, et une position ($\alpha_{FL}$) de la fausse lumière par rapport à la vraie lumière.

6. Méthode selon l'une quelconque des revendications 3 à 5, dans laquelle, dans ladite étape de calcul (22), chaque valeur de similitude est calculée selon

$$p(AS_i|\mathbf{Z})=\frac{p(AS_i)}{Vol(\mathbf{R}_i)}\int_{\mathbf{R}_i}p(\mathbf{Z}|\mathbf{x}_i,AS_i)dV_{\mathbf{x}_i}$$

préférentiellement selon

$$p(AS_i|\mathbf{Z})=\frac{p(AS_i)}{Vol(\mathbf{R}_i)}\int_{\mathbf{R}_i}\prod_{\substack{j=1,\dots,J\\k=1,\dots,K}}\frac{1}{\sqrt{2\pi\Delta_{i,j,k}^2}}\exp\left(-\frac{\left(Z_j(t_k)-f_{i,j}(t_k,\mathbf{x}_i)\right)^2}{2\Delta_{i,j,k}^2}\right)dV_{\mathbf{x}_i}$$

avec

$p(AS_i|\mathbf{Z})$ la valeur de similitude calculée du $i^e$ état aortique d'après les impédances Z détectées,

$p(AS_i)$ une probabilité a priori prédéterminée pour le $i^e$ état aortique,

$\mathbf{R}_i$ un ensemble de valeurs prédéterminé des paramètres aortiques $\mathbf{x}_i$ du $i^e$ état aortique,

$Vol(\mathbf{R}_i)$ un hypervolume étendu par ledit ensemble de valeurs prédéterminé,

$p(\mathbf{Z}|\mathbf{x}_i,AS_i)$ le produit de fonctions de probabilité,

$dV_{\mathbf{x}_i}$ un élément d'hypervolume,

$\int_{\mathbf{R}_i}$ une intégration sur l'ensemble de valeurs prédéter-miné,

$\prod_{\substack{j=1,\dots,J\\k=1,\dots,K}}$ un produit sur la totalité des J ensembles d'électrodes d'ICG et la totalité des K temps de détection,

$Z_j(t_k)$ l'impédance détectée pour le $j^e$ ensemble ($4_1$, $4_2$) d'électrodes d'ICG ($5_{j,in}$, $5_{j,se}$) au $k^e$ temps de détection $t_k$,

$f_{i,j}(t_k)$ l'impédance de référence pour le $i^e$ état aortique et le $j^e$ ensemble ($4_j$) d'électrodes d'ICG ($5_{j,in}$, $5_{j,se}$) au $k^e$

temps de détection $t_k$, et

$\Delta_{i,j,k}$ une incertitude estimée quant à la détection par le $j^e$ ensemble $(4_j)$ d'électrodes d'ICG $(5_{j,in}, 5_{j,se})$ et/ou quant à l'impédance de référence $f_{i,j}$ pour le $i^e$ état aortique et le $j^e$ ensemble $(4_j)$ d'électrodes d'ICG $(5_{j,in}, 5_{j,se})$ au $k^e$ temps de détection $t_k$.

**7.** Méthode selon l'une quelconque des revendications 3 à 6, dans laquelle, dans ladite étape de calcul (22), chaque impédance de référence est calculée au moyen du processeur (8) à partir du modèle de substitution qui décrit l'impédance de référence par

$$f_{i,j}(\mathbf{x}_i, t_k) = f_{i,j}(\mathbf{x}_i; \mathbf{c}_i(t_k)) = \sum_{q=1,\ldots,Q} c_{i,j,q}(t_k) \cdot \phi_q(\mathbf{x}_i)$$

avec

$f_{i,j}(\mathbf{x}_i, t_k)$ l'impédance de référence calculée pour le $i^e$ état aortique et le $j^e$ ensemble $(4_j)$ d'électrodes d'ICG $(5_{j,in}, 5_{j,se})$ au $k^e$ temps de détection $t_k$ en fonction des paramètres aortiques $\mathbf{x}_i$ du $i^e$ état aortique,

$\phi_q(\mathbf{x}_i)$ un polynôme multivarié dans les paramètres aortiques $\mathbf{x}_i$ du $i^e$ état aortique, où les polynômes sont indexés par q,

$c_{i,j,q}(t_k)$ un coefficient de développement pour le $i^e$ état aortique, le $j^e$ ensemble $(4_j)$ d'électrodes d'ICG $(5_{j,in}, 5_{j,se})$ et le $q^e$ polynôme au $k^e$ temps de détection,

$\mathbf{c}_i$ l'ensemble de la totalité des coefficients de développement pour le $i^e$ état aortique, et

$\sum_{q=1,\ldots,Q}$ une somme sur la totalité des Q polynômes multivariés.

**8.** Méthode selon l'une quelconque des revendications 3 à 7, dans laquelle les objets de référence sont des modèles de méthode des éléments finis, MEF, de thoraces (20) et la méthode (11) comprend en outre, avant ladite étape de stockage (21), une étape de génération (24), au moyen du processeur (8), de chaque modèle de substitution par :

la détermination (25), pour chacun des objets de référence (20), des valeurs des paramètres aortiques $(R_{TL}, R_{FL}, \alpha_{FL})$ de l'état aortique de cet objet de référence (20) ;

l'application (26), pour chaque ensemble $(4_1, 4_2)$ d'électrodes d'ICG $(5_{1,in}, 5_{1,se}; 5_{2,in}, 5_{2,se})$ et chacun des objets de référence (20), des électrodes d'ICG $(5_{1,in}, 5_{1,se}; 5_{2,in}, 5_{2,se})$ sur le thorax de cet objet de référence au niveau des emplacements respectifs $(14_{1,in}, 14_{1,se}; 14_{2,in}, 14_{2,se})$ et la quantification (27), au moyen des électrodes d'ICG $(5_{1,in}, 5_{1,se}; 5_{2,in}, 5_{2,se})$ appliquées, d'une impédance au cours du temps ; et

le calcul (28), au moyen du processeur (8) à partir des valeurs déterminées des paramètres aortiques et des impédances quantifiées, des coefficients de développement.

**9.** Méthode selon la revendication 8, dans laquelle les coefficients de développement sont calculés selon

$$c_{i,j,q}(t_k) = \sum_l \left( \left(\mathbf{U}_i^T \cdot \mathbf{U}_i\right)^{-1} \cdot \mathbf{U}_i^T \right)_{ql} Z_j^l(t_k)$$

avec

$c_{i,j,q}(t_k)$ le coefficient de développement pour le $i^e$ état aortique, le $j^e$ ensemble $(4_j)$ d'électrodes d'ICG $(5_{j,in}, 5_{j,se})$ et le $q^e$ polynôme au $k^e$ temps de détection $t_k$,

$\mathbf{U}_i = \phi_q(\mathbf{v}_i^l)$ une matrice pour le $i^e$ état aortique formé par l'évaluation du $q^e$ polynôme multivarié au niveau des valeurs $\mathbf{v}_i^l$ déterminées des paramètres aortiques $\mathbf{x}_i$ du $l^e$ objet de référence (20) ayant le $i^e$ état aortique, et

$Z_j^l(t_k)$ l'impédance quantifiée du $l^e$ objet de référence (20) dans le $j^e$ ensemble $(4_j)$ d'électrodes d'ICG $(5_{j,in}, 5_{j,se})$ au $k^e$ temps de détection $t_k$.

**10.** Dispositif de traitement pour la détermination d'un état aortique d'un patient (2), le dispositif de traitement (6) ayant

une mémoire (7) qui est conçue pour recevoir (19), depuis au moins deux ensembles $(4_1, 4_2)$ différents d'électrodes de cardiographie d'impédance, ICG, $(5_{1,in}, 5_{1,se}; 5_{2,in}, 5_{2,se})$ pouvant être connectées audit dispositif de traitement (6) et conçues pour être placées dans une configuration d'électrodes tétrapolaire aux emplacements ($14_{1,in}$, $14_{1,se}$ ; $14_{2,in}$, $14_{2,se}$) respectifs sur le thorax du patient (15), une impédance ($Z_1$, $Z_2$) détectée au cours du temps par cet ensemble ($4_1$, $4_2$) d'électrodes d'ICG ($5_{1,in}$, $5_{1,se}$ ; $5_{2,in}$, $5_{2,se}$), et pour stocker (21), pour chacun parmi un ensemble prédéterminé d'états aortiques et chaque ensemble ($4_1$, $4_2$) d'électrodes d'ICG ($5_{1,in}$, $5_{1,se}$ ; $5_{2,in}$, $5_{2,se}$), un modèle de substitution généré pour une pluralité d'objets de référence (20) ayant cet état aortique, lequel modèle de substitution décrit une impédance de référence au cours du temps ; et
un processeur (8) connecté à ladite mémoire (7) et conçu pour calculer (22), pour chaque état aortique de l'ensemble ($4_1$, $4_2$), une valeur de similitude depuis un produit de fonctions de probabilité, chacune dépendant d'une différence entre l'impédance ($Z_1$, $Z_2$) détectée pour un ensemble ($4_1$, $4_2$) respectif d'électrodes d'ICG ($5_{1,in}$, $5_{1,se}$ ; $5_{2,in}$, $5_{2,ie}$) et l'impédance de référence pour cet état aortique et le même ensemble ($4_1$, $4_2$) d'électrodes d'ICG ($5_{1,in}$, $5_{1,se}$ ; $5_{2,in}$, $5_{2,se}$), et pour déterminer (23) l'état aortique avec la valeur de similitude calculée la plus élevée en tant qu'état aortique du patient.

11. Dispositif de traitement selon la revendication 10, dans lequel chaque impédance de référence est un développement des fonctions de base qui dépendent de paramètres aortiques prédéfinis ($R_{TL}$, $R_{FL}$, $\alpha_{FL}$) de l'état aortique, et dans lequel le processeur (8) est conçu pour réaliser ladite étape de calcul (22) par la marginalisation du produit de fonctions de probabilité par rapport aux paramètres aortiques ($R_{TL}$, $R_{FL}$, $\alpha_{FL}$).

12. Dispositif de traitement selon la revendication 11, dans lequel le processeur (8) est conçu pour calculer la valeur de similitude de chaque état aortique selon

$$p\big(AS_i \big| \mathbf{Z}\big) = \frac{p(AS_i)}{Vol(\mathbf{R}_i)} \int_{\mathbf{R}_i} p\big(\mathbf{Z}\big|\mathbf{x}_i, AS_i\big) dV_{\mathbf{x}_i}$$

préférentiellement selon

$$p\big(AS_i \big| \mathbf{Z}\big) = \frac{p(AS_i)}{Vol(\mathbf{R}_i)} \int_{\mathbf{R}_i} \prod_{\substack{j=1,...,J \\ k=1,...,K}} \frac{1}{\sqrt{2\pi\Delta_{i,j,k}^2}} \exp\left(-\frac{\big(Z_j(t_k) - f_{i,j}(\mathbf{x}_i, t_k)\big)^2}{2\Delta_{i,j,k}^2}\right) dV_{\mathbf{x}_i}$$

avec

$p(AS_i|Z)$ la valeur de similitude calculée du $i^e$ état aortique d'après les impédances Z détectées,
$p(AS_i)$ une probabilité a priori prédéterminée pour le $i^e$ état aortique $AS_i$,
$\mathbf{R}_i$ un ensemble de valeurs prédéterminé des paramètres aortiques $\mathbf{x}_i$ du $i^e$ état aortique,
$Vol(\mathbf{R}_i)$ un hypervolume étendu par ledit ensemble de valeurs prédéterminé,
$p(\mathbf{Z}|\mathbf{x}_i, AS_i)$ le produit de fonctions de probabilité,
$dV_{\mathbf{x}_i}$ un élément d'hypervolume,

$\displaystyle\int_{\mathbf{R}_i}$ une intégration sur l'ensemble de valeurs prédéterminé,

$\displaystyle\prod_{\substack{j=1,...,J \\ k=1,...,K}}$ un produit sur la totalité des J ensembles d'électrodes d'ICG et la totalité des K temps de détection,

$Z_j(t_k)$ l'impédance détectée pour le $j^e$ ensemble ($4_j$) d'électrodes d'ICG ($5_{j,in}$, $5_{j,se}$) au $k^e$ temps de détection $t_k$,
$f_{i,j}(\mathbf{x}_i, t_k)$ l'impédance de référence pour le $i^e$ état aortique et le $j^e$ ensemble ($4_j$) d'électrodes d'ICG ($5_{j,,in}$, $5_{j,se}$) au $k^e$ temps de détection $t_k$ en fonction des paramètres aortiques $\mathbf{x}_i$ du $i^e$ état aortique, et
$\Delta_{i,j,k}$ une incertitude estimée quant à la détection par le $j^e$ ensemble ($4_j$) d'électrodes d'ICG ($5_{j,in}$, $5_{j,se}$) et/ou quant à l'impédance de référence $f_{i,j}$ pour le $i^e$ état aortique et le $j^e$ ensemble ($4_j$) d'électrodes d'ICG ($5_{j,in}$, $5_{j,se}$) au $k^e$ temps de détection $t_k$.

13. Dispositif de traitement selon la revendication 11 ou 12, dans lequel le processeur (8) est en outre conçu pour calculer chaque impédance de référence à partir du modèle de substitution qui décrit l'impédance de référence par

$$f_{i,j}(\mathbf{x}_i, t_k) = f_{i,j}(\mathbf{x}_i; \mathbf{c}_i(t_k)) = \sum_{q=1,\ldots,Q} c_{i,j,q}(t_k) \cdot \phi_q(\mathbf{x}_i)$$

avec

$f_{i,j}(\mathbf{x}_i, t_k)$ l'impédance de référence pour le $i^e$ état aortique et le $j^e$ ensemble ($4_j$) d'électrodes d'ICG ($5_{j,in}$, $5_{j,se}$) au $k^e$ temps de détection $t_k$ en fonction des paramètres aortiques $\mathbf{x}_i$ du $i^e$ état aortique,

$\phi_q(\mathbf{x}_i)$ un polynôme multivarié dans les paramètres aortiques $\mathbf{x}_i$ du $i^e$ état aortique, où les polynômes sont indexés par q,

$c_{i,j,q}(t_k)$ un coefficient de développement pour le $i^e$ état aortique, le $j^e$ ensemble ($4_j$) d'électrodes d'ICG ($5_{j,in}$, $5_{j,se}$) et le $q^e$ polynôme au $k^e$ temps de détection $t_k$,

$\mathbf{c}_i$ l'ensemble de la totalité des coefficients de développement pour le $i^e$ état aortique, et

$\displaystyle\sum_{q=1,\ldots,Q}$ une somme sur la totalité des Q polynômes multiva-riés.

14. Système de mesure comprenant le dispositif de traitement (6) selon l'une quelconque des revendications 10 à 13 et lesdits au moins deux ensembles ($4_1$, $4_2$) différents d'électrodes d'ICG ($5_{1,in}$, $5_{1,se}$ ; $5_{2,in}$, $5_{2,se}$), dans lequel chaque ensemble ($4_1$, $4_2$) d'électrodes d'ICG ($5_{1,in}$, $5_{1,se}$ ; $5_{2,in}$, $5_{2,se}$) est conçu pour être placé (16) dans la configuration d'électrodes tétrapolaire au niveau des emplacements ($14_{1,in}$, $14_{1,se}$ ; $14_{2,in}$, $14_{2,se}$) respectifs sur le thorax du patient (15), pour détecter (17) ladite impédance ($Z_1$, $Z_2$) au cours du temps et pour transmettre (18) l'impédance ($Z_1$, $Z_2$) détectée à la mémoire (7).

**Fig. 1**

**Fig. 2**

**Fig. 3**

**Fig. 4**

**Fig. 5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **V. BADELI**. Electrode positioning to investigate the changes of the thoracic bioimpedance caused by aortic dissection - a simulation study. *Journal of electical bioimpedance*, 2020, vol. 11, 38-48

- **C. PICHLER**. Bayesian source separation of electrical bioimpedance signals. *Biomedical Signal Processing and Control*, 2021, vol. 67 (102541)